# EUROPEAN PATENT APPLICATION

(11) **EP 4 120 219 A1**
(43) Date of publication of application: **18.01.2023**
(21) Application number: 21767509.9
(22) Date of filing: 12.03.2021
(51) Int. Cl.: G08G 1/16, G01C 21/26, G08G 1/01, G08G 1/137

(54) **POSITION-CORRESPONDING EMOTION EVALUATION DEVICE AND POSITION-CORRESPONDING EMOTION EVALUATION SYSTEM**

(30) Priority: 13.03.2020 WO PCT/JP2020/011253
(71) Applicant: Yamaha Hatsudoki Kabushiki Kaisha, Iwata-shi, Shizuoka 438-8501 (JP)
(72) Inventor: SAMESHIMA Akira, Iwata-shi, Shizuoka 438-8501 (JP); INOUE Shinichi, Iwata-shi, Shizuoka 438-8501 (JP)
(74) Representative: Zinkler, Franz
(86) International application number: PCT/JP2021/010192
(87) International publication number: WO 2021/182628

(57) **Abstract**

Provided is a position-corresponding-emotion-evaluation device that can effectively use information on an emotion, while suppressing an increase in the processing load of hardware. The position-corresponding-emotion-evaluation device includes: a biological-information-acquisition unit 2 for acquiring biological information Bi on at least a heartbeat of a target person, a position-information-acquisition unit 3 for acquiring position information Pi on a position where the biological-information-acquisition unit 2 has acquired the biological information Bi, an emotional-information-generating unit 4 for generating emotional information Ei on an emotion of the target person based on the biological information Bi, a position evaluation unit 5 for acquiring the emotional information Ei and the position information Pi and generating position-corresponding-emotion-associated information Vi1 in which the acquired position information Pi related to the biological information Bi used for generation of the acquired emotional information Ei, and the emotional information Ei are associated with each other, and an output unit 6 for outputting the position-corresponding-emotion-evaluation information Vi1.

## Description

### TECHNICAL FIELD

The present teaching relates to a position-corresponding-emotion-evaluation device and a position-corresponding-emotion-evaluation system.

### BACKGROUND ART

Along with widespread use of navigational devices, technologies for displaying various information, such as information on roads, accidents and stores, on map information, have been proposed. Also proposed are technologies for estimating a body condition and a state of mind of a target person based on biological information of the target person when the target person receives an external event that might affect the state of mind of the target person.

A risk level determining device disclosed in Patent Document 1 acquires information on the movement of a vehicle, such as a position and a speed, by a movement information acquisition unit, and also acquires biological information such as a heartbeat of an occupant of the vehicle by a biological information acquisition unit. The risk level determining device determines a section at which the biological information greatly changes as a high-risk section, based on the acquired movement information and biological information.

An emotion estimation apparatus disclosed in Patent Document 2 causes a test subject to input information indicating his/her own current emotion on an emotion input device, and also measures, for example, biological information indicating human activity correlated with human emotions by a measurement device incorporated in, for example, a wearable terminal. The emotion estimation apparatus generates regression equations for estimating an emotion by multiple regression analysis by using the input information indicating the emotion of the test subject as supervisory data, and feature quantities of the measured biological information, for example, as variables. The emotion estimation apparatus estimates emotional changes of the test subject based on the measured biological information using the generated regression equations.

A vehicle driving support system disclosed in Patent Document 3 has an artificial intelligence which learns a general driver model to be applied to a plurality of drivers based on driving data of the drivers and updates it. The artificial intelligence analyzes an emotional state of a driver based on driver state data including heart rate data of the driver. The artificial intelligence analyzes a relevance between the analyzed emotional state and a vehicle state including vehicle speed, longitudinal acceleration, and lateral acceleration, for example. Furthermore, the artificial intelligence analyzes a new vehicle state that causes the emotional state. When the new vehicle state that causes the emotional state is detected, the artificial intelligence generates a new relevance model based on the new vehicle state.

### CITATION LIST

### PATENT DOCUMENT

Patent Document 1: Japanese Patent Application Publication No. 2018-181386
Patent Document 2: Japanese Patent Application Publication No. 2018-102617
Patent Document 3: Japanese Patent Application Publication No. 2018-169706

### SUMMARY OF INVENTION

### TECHNICAL PROBLEM

The risk level determining device disclosed in Patent Document 1 can suppress occurrence of accidents and trouble by conveying information on high-risk places to an occupant.

The emotion estimation apparatus disclosed in Patent Document 2 estimates in real time, based on biological information of a test subject, for example, and on regression equations, emotional changes of the test subject at that time. Accordingly, the emotion estimation apparatus can estimate the emotional changes of the test subject without monitoring external events, such as environmental conditions around the test subject. In addition, the emotion estimation apparatus disclosed in Patent Document 2 does not require a configuration for monitoring the external events, and thus is applicable to a wide range of applications without increasing hardware load.

The emotion estimation apparatus does not monitor the external events when the apparatus estimates that the emotion has occurred on the test subject. Therefore, the emotion estimation apparatus does not allow a user to grasp what external events affect the test subject and generate the emotion. The emotion that occurs on the test subject is closely related to the external events. Thus, the user cannot determine whether the information on the emotion output by the emotion estimation apparatus is appropriate for the user's purpose of use. That is, there is a possibility that the user cannot effectively use the information on the emotion output by the emotion estimation apparatus. On the other hand, estimating an emotion of the test subject while monitoring external events that affect the emotion of the test subject entails an increase in the processing load of hardware.

The vehicle driving support system disclosed in Patent Document 3 acquires and analyzes, for example, heart rate data and position information of a driver in order to provide vehicle control processing most appropriate for driving characteristics of the driver. The vehicle driving support system also analyzes an emotional state of the driver based on the heart rate data, for example. In a case where the vehicle driving support system estimates that the driver feels a low level of pleasure, the system recommends to the driver the places that have been registered as those making the driver feel pleasant, within a predetermined range of distance from the current position. In this manner, the vehicle driving support system performs vehicle control processing and navigation based on the emotional state of the driver. In the vehicle driving support system, however, there is no recitation for associating the emotional state of the driver and the position of the driver. Therefore, there have been demands for effective utilization of information on an emotion by associating an emotion and a position. In the hardware for processing the information on an emotion, the capacity for simultaneous parallel processing of various information in addition to the information on an emotion is required. Therefore, there have been demands for a configuration capable of effectively using the information on an emotion, while suppressing an increase in the processing load of hardware.

It is an object of the present teaching to provide a position-corresponding-emotion-evaluation device and a position-corresponding-emotion-evaluation system, capable of effectively using information on an emotion, while suppressing an increase in the processing load of hardware.

### SOLUTION TO PROBLEM

The inventors of the present teaching studied a configuration of a device capable of effectively using information on an emotion, while suppressing an increase in the processing load of hardware. Through an intensive study, the inventors arrived at the configuration of a position-corresponding-emotion-evaluation device using the information on an emotion as described below.

A position-corresponding-emotion-evaluation device according to one embodiment of the present teaching comprises: a storage unit configured to store biological information on at least a heartbeat of a target person, and position information on a position where the biological information has been measured; and a control unit configured to acquire and store in the storage unit, the biological information and the position information, have a criterion for generating emotional information on an emotion based on the biological information, generate the emotional information based on the biological information, based on the criterion, and output the position information and the emotional information. The control unit is configured to generate position-corresponding-emotion-associated information in which the position information on the position where the biological information has been measured, and the emotional information on the emotion of the target person generated using the biological information measured at the position are associated, and output the emotional information associated with the position information, or the position information associated with the emotional information, from the generated position-corresponding-emotion-associated information.

In the configuration described above, the position-corresponding-emotion-evaluation device stores biological information on at least a heartbeat of a target person, and position information on a position where the biological information has been measured by the storage unit. The position-corresponding-emotion-evaluation device generates emotional information on an emotion of the target person based on the biological information on at least the heartbeat of the target person by the control unit. The position-corresponding-emotion-evaluation device acquires, by the control unit, the position information on the place where the biological information on at least the heartbeat of the target person has been acquired. Furthermore, the position-corresponding-emotion-evaluation device generates, by the control unit, position-corresponding-emotion-associated information in which the position information and the emotional information are associated. In this manner, the position-corresponding-emotion-evaluation device generates the emotional information based on the biological information on at least the heartbeat of the target person including no external events, so that the processing load of hardware can be reduced.

The position-corresponding-emotion-evaluation device outputs the emotional information associated with the position information, or the position information associated with the emotional information, from the generated position-corresponding-emotion-associated information. In this manner, the position-corresponding-emotion-evaluation device associates the position information and the emotional information that can be easily acquired through the use of existing technologies and facilities, so that the device can evaluate the position information by an index concerning emotion. That is, the position-corresponding-emotion-evaluation device can convert the position information merely indicating a place where the target person is located into value-added information indicating what the place means for the target person. Furthermore, the position-corresponding-emotion-evaluation device adds the position information to the emotional information, which is conducive to an increase in applications of the emotional information. That is, the position-corresponding-emotion-evaluation device can convert the emotional information merely indicating an emotion of the target person into value-added information indicating what external events caused the emotion of the target person. This allows the position-corresponding-emotion-evaluation device to effectively use the information on an emotion, while suppressing an increase in the processing load of the hardware.

In another aspect, the position-corresponding-emotion-evaluation device according to the present teaching preferably includes the following configuration. The emotion includes a plurality of types of emotions, and the emotional information is emotional information on an emotion selected from the plurality of the types of the emotions.

As described above, the control unit of the position-corresponding-emotion-evaluation device generates the emotional information on an emotion selected from the plurality of types of emotions, based on the biological information on at least a heartbeat of the target person. The position-corresponding-emotion-evaluation device selects one emotion from the plurality of emotions, and thereby can flexibly respond to changes in emotion of the target person. This allows the position-corresponding-emotion-evaluation device to effectively use the information on an emotion, while suppressing an increase in the processing load of the hardware.

In another aspect, the position-corresponding-emotion-evaluation device according to the present teaching preferably includes the following configuration. The emotional information includes information on degree of an emotion.

As described above, the position-corresponding-emotion-evaluation device generates, by the control unit, the emotional information including degree of an emotion based on biological information on at least a heartbeat of the target person. The position-corresponding-emotion-evaluation device selects the degree of an emotion, and thereby can flexibly respond to changes in emotion of the target person. This allows the position-corresponding-emotion-evaluation device to effectively use the information on an emotion, while suppressing an increase in the processing load of the hardware.

The position-corresponding-emotion-evaluation device according to one embodiment of the present teaching preferably includes the following configuration. The control unit is configured to calculate a moving speed of the target person based on the position information and time at which the position information has been acquired. The control unit is configured to generate the position-corresponding-emotion-associated information based on the calculated moving speed. The control unit is configured to output the emotional information associated with the position information, or the position information associated with the emotional information, from the position-corresponding-emotion-associated information generated based on the moving speed.

As described above, the position-corresponding-emotion-evaluation device generates different emotional information corresponding to different moving speeds, with the position and the time included in the acquired position information as indexes. That is, the position-corresponding-emotion-evaluation device generates the emotional information by using a different index in accordance with each different means of transportation that is an external event affecting an emotion of the target person. The position-corresponding-emotion-evaluation device can evaluate the position information by the emotional information reflecting the difference in the means of transportation. This allows the position-corresponding-emotion-evaluation device to effectively use the information on an emotion, taking into consideration the influence of a moving speed (a means of transportation) on an emotion, without the processing load applied to the hardware.

The position-corresponding-emotion-evaluation device according to one embodiment of the present teaching preferably includes the following configuration. The control unit is configured to generate the position-corresponding-emotion-associated information based on the time at which the position information has been acquired. The control unit is configured to output the emotional information associated with the position information, or the position information associated with the emotional information, from the position-corresponding-emotion-associated information generated based on the time.

The position-corresponding-emotion-evaluation device generates different emotional information corresponding to different time intervals arbitrarily defined, such as seasons and time frames, with the time included in the acquired position information as an index. That is, the position-corresponding-emotion-evaluation device generates the emotional information, by using a different index in accordance with a change in surrounding environment according to a difference in time that is an external event affecting the emotion of the target person. The position-corresponding-emotion-evaluation device can evaluate the position information by the emotional information reflecting the difference in season and time frame, for example. This allows the position-corresponding-emotion-evaluation device to effectively use the information on an emotion, taking into consideration the influence of a season and a time frame on an emotion, without the processing load applied to the hardware.

The position-corresponding-emotion-evaluation device according to one embodiment of the present teaching preferably includes the following configuration. The control unit is configured to acquire weather information based on the position information. The control unit is configured to generate the position-corresponding-emotion-associated information based on the weather information. The control unit is configured to output the emotional information associated with the position information, or the position information associated with the emotional information, from the position-corresponding-emotion-associated information generated based on the weather information.

As described above, the position-corresponding-emotion-evaluation device acquires, based on the acquired position information, weather information at a position corresponding to the position information. Furthermore, the position-corresponding-emotion-evaluation device generates the emotional information with the weather information as an index. That is, the position-corresponding-emotion-evaluation device generates the emotion-corresponding-emotional information by using, as the index, the weather information that is an external event affecting an emotion of the target person. The position-corresponding-emotion-evaluation device can evaluate the position information, by using the emotion reflecting the difference in weather condition as an index. This allows the position-corresponding-emotion-evaluation device to effectively use the information on an emotion, taking into consideration the influence of weather conditions on an emotion, without the processing load applied to the hardware.

The position-corresponding-emotion-evaluation device according to one embodiment of the present teaching preferably includes the following configuration. The control unit is configured to output the emotional information in form of an emotion label that is obtained by converting the emotional information into at least one of a numerical value, a mark, a figure or a color.

As described above, the emotional information is converted into at least one of a numerical value, a mark, a figure or a color that is easy to visually recognize, so that the emotional information can be easily displayed on a map, a graph and a chart in a manner to be easily visually recognized. This allows the position-corresponding-emotion-evaluation device to effectively use the information on an emotion, while suppressing an increase in the processing load of the hardware.

A position-corresponding-emotion-evaluation system according to one embodiment of the present teaching comprises: a storage unit configured to store pieces of biological information on at least heartbeats of target persons, and pieces of position information on positions where the pieces of the biological information have been measured, the pieces of the biological information and the pieces of the position information having been acquired from the target persons; and a control unit configured to acquire, and store in the storage unit, the pieces of the biological information and the pieces of the position information, have a criterion for generating pieces of emotional information on emotions based on the pieces of the biological information, generate the pieces of the emotional information based on the pieces of the biological information, based on the criterion, and output the pieces of the position information and the pieces of the emotional information. The control unit is configured to generate pieces of position-corresponding-emotion-associated information in which the pieces of the position information on the positions where the pieces of the biological information have been measured, and the pieces of the emotional information on the emotions of the target persons generated using the pieces of the biological information measured at the positions, are associated. Furthermore, the control unit is configured to output the pieces of the emotional information associated with the pieces of the position information, or the pieces of the position information associated with the pieces of the emotional information, from the generated pieces of the position-corresponding-emotion-associated information.

In the configuration described above, the position-corresponding-emotion-evaluation system stores the pieces of the biological information on at least the heartbeats of the target persons, and the pieces of the position information on the positions where the pieces of the biological information have been measured. The position-corresponding-emotion-evaluation device generates the pieces of the position-corresponding-emotion-associated information based on the stored pieces of the emotional information on the emotions of the target persons and the stored pieces of the position information on the positions where the pieces of the biological information of the target persons have been measured. The position-corresponding-emotion-evaluation system stores the pieces of the position-corresponding-emotion-associated information by the storage unit. That is, the position-corresponding-emotion-evaluation system has a collection of the position-corresponding-emotion-associated information (big data). The position-corresponding-emotion-evaluation system generates the pieces of the emotional information associated with the pieces of the position information, or the pieces of the position information associated with the pieces of the emotional information based on the collection of the position-corresponding-emotion-associated information. Thus, the position-corresponding-emotion-evaluation system can evaluate the position information by the indexes excluding the influence of each individual's personality, such as character and taste of each individual, from the emotional information. This allows the position-corresponding-emotion-evaluation system to effectively use the information on an emotion, while suppressing an increase in the processing load of the hardware.

The terminology used herein is for the purpose of describing particular embodiments only and is not intended to be limiting of the invention.

As used herein, the term "and/or" includes any and all combinations of one or more of the associated listed items.

It will be further understood that the terms "including," "comprising" or "having" and variations thereof when used in this specification, specify the presence of stated features, steps, elements, components, and/or their equivalents, but do not preclude the presence or addition of one or more steps, operations, elements, components, and/or groups thereof.

It will be further understood that the terms "mounted," "connected," "coupled," and/or their equivalents are used broadly and encompass both direct and indirect mounting, connecting and coupling. Further, "connected" and "coupled" are not restricted to physical or mechanical connections or couplings, and can include connections or couplings, whether direct or indirect.

Unless otherwise defined, all terms (including technical and scientific terms) used herein have the same meaning as commonly understood by one having ordinary skill in the art to which this invention belongs.

It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

In describing the invention, it will be understood that a number of techniques and steps are disclosed. Each of these has individual benefit and each can also be used in conjunction with one or more, or in some cases all, of the other disclosed techniques.

Accordingly, for the sake of clarity, this description will refrain from repeating every possible combination of the individual steps in an unnecessary fashion. Nevertheless, the specification and claims should be read with the understanding that such combinations are entirely within the scope of the invention.

Embodiments of a position-corresponding-emotion-evaluation device according to the present invention will be herein described.

In the following description, numerous specific details are set forth in order to provide a thorough understanding of the present invention. It will be evident, however, to one skilled in the art that the present invention may be practiced without these specific details.

The present disclosure is to be considered as an exemplification of the invention, and is not intended to limit the invention to the specific embodiments illustrated by the figures or description below.

### [Target Person]

A target person herein is a person targeted for acquisition of biological information on at least a heartbeat and position information on a place by a position-corresponding-emotion-evaluation device. The position-corresponding-emotion-evaluation device acquires biological information on at least a heartbeat of a target person. The position-corresponding-emotion-evaluation device also acquires position information on a place where the biological information on at least the heartbeat of the target person has been acquired.

### [Information on Target Person]

Information on a target person herein includes information on the target person, such as gender, age, physical features that are, for example, height and weight, character, likes and tastes of the target person. The information on a target person is input in advance on the position-corresponding-emotion-evaluation device by the target person him/herself. The information on a target person does not include personal information specifying the target person, such as name and address.

### [External Event]

An external event herein includes an event, a phenomenon and a situation that provide a target person with, for example, information and stimuli from outside, such as environment around the location of the target person and work being performed by the target person. Examples of the external event include a state where a target person is moving by a leaning vehicle, a state where the target person is moving on foot, and a state where the target person is seeing a photograph of an animal.

### [Test Subject]

A test subject herein is a person targeted for storage of biological information on a heartbeat and emotional information on a self-assessed emotion for a specific external event in a training database in order to create training data for a classifier that generates emotional information. The classifier generates training data for classifying an emotion of a target person, by using pieces of biological information on heartbeats and pieces of emotional information on self-assessed emotions acquired from a plurality of test subjects.

### [Biological Information]

Biological information herein is various physiological and anatomical information from a target person (a living body). The biological information includes information input in the living body and information output from the living body to regulate the living body of the target person. Examples of the biological information include information on a heart rate, heart tones, a heartbeat waveform, a cardiac cycle, a change in heart rate, blood pressure, a high-frequency fluctuation component (HF), a low-frequency fluctuation component (LF), triaxial acceleration, body surface temperature, brain waves, a respiratory rate, a condition of pupils, myoelectric potential, blood components, expiratory volume, and expiratory components of the target person. The biological information is measured by, for example, a heart rate sensor, an acceleration sensor, and a temperature sensor that are controlled by a control unit provided in underclothing worn by the target person, for example.

### [Biological Information on Heartbeat of Target Person]

Biological information on a heartbeat of a target person herein includes, of the biological information, information on a heart rate, a heartbeat waveform, a cardiac cycle, a change in heart rate, blood pressure, a high-frequency fluctuation component (HF), and a low-frequency fluctuation component (LF), of the target person, for example. The biological information on a heartbeat is measured by a heart rate sensor that is a biological-information-acquisition unit provided in underclothing worn by the target person, for example.

### [Position Information on Place Where Target Person Is Located]

Position information on a place where a target person is located herein includes, for example, information on latitude, longitude, altitude, time of measurement, environment and weather concerning the place where the target person is located. The position information on a position is calculated by, for example, a receiver for a global navigation satellite system, a beacon, a gyroscope sensor, and a direction sensor that are controlled by a control unit provided in, for example, a portable terminal carried by the target person.

### [Emotional Information on Emotion of Target Person]

Emotional information on an emotion of a target person herein includes information on emotions, such as delight, anger, sorrow, and pleasure of the target person. The emotional information on an emotion of a target person includes a state where the target person feels happy, a state where the target person feels relaxed, a state where the target person feels angry, and a state where the target person feels sad. The emotional information on an emotion of a target person is generated by a control unit provided in, for example, a portable terminal carried by the target person or a server that acquires the biological information on a heartbeat.

### [Mind-and-Body-State Information on Mind and Body State of Target Person]

Mind-and-body-state information on a mind and body state of a target person herein includes information on the influence exerted on the body by an emotion of the target person. The mind-and-body-state information on a mind and body state of a target person includes, for example, a state where the target person feels a sense of exaltation, a state where the target person feels relaxed, a state where the target person feels tense, and a state where the target person feels sleepy. The mind-and-body-state information on a mind and body state of a target person is generated by a control unit provided in, for example, a portable terminal carried by the target person or a server that acquires the biological information on a heartbeat.

### [Degree]

Degree herein is stepwise expression of the strength of an emotion. For example, degree of comfort is expressed by stepwise assignment of standard emotions on comfort, in a range from a standard emotion in which an emotion on comfort is predominant and the most strongly exhibited, to a standard emotion in which an emotion on comfort is predominant but the least exhibited (not uncomfortable, but not feel comfortable).

### ADVANTAGEOUS EFFECTS OF INVENTION

According to one embodiment of the present teaching, it is possible to effectively use information on an emotion, while suppressing an increase in the processing load of hardware.

### BRIEF DESCRIPTION OF DRAWINGS

[FIG. 1] FIG. 1 is a schematic view of an overall configuration of a position-corresponding-emotion-evaluation device according to a first embodiment of the present teaching.
[FIG. 2] FIG. 2 is a schematic view of a mode of display of position-corresponding-emotion-evaluation information by the position-corresponding-emotion-evaluation device according to the first embodiment of the present teaching.
[FIG. 3] FIG. 3 is a graph showing classification indexes of emotional information in the position-corresponding-emotion-evaluation device according to the first embodiment of the present teaching.
[FIG. 4] FIG. 4 is a graph showing classification indexes of mind-and-body-state information in the position-corresponding-emotion-evaluation device according to the first embodiment of the present teaching.
[FIG. 5] FIG. 5 is a control flow diagram showing control of the position-corresponding-emotion-evaluation device according to the first embodiment of the present teaching.
[FIG. 6] FIG. 6 is a schematic view of an overall configuration of a position-corresponding-emotion-evaluation device according to a second embodiment of the present teaching.
[FIG. 7] FIG. 7 is a schematic view of a configuration of the position-corresponding-emotion-evaluation device according to the second embodiment of the present teaching, which is worn by a target person.
[FIG. 8] FIG. 8 is a schematic view of a display state of position-corresponding-emotion-evaluation information based on emotional information and body state information in the position-corresponding-emotion-evaluation device according to the second embodiment of the present teaching.
[FIG. 9] FIG. 9 is a schematic view of an overall configuration of a position-corresponding-emotion-evaluation system according to a third embodiment of the present teaching.
[FIG. 10] FIG. 10 is a schematic view of an overall configuration of a position-corresponding-emotion-evaluation device according to a fourth embodiment of the present teaching.
[FIG. 11] FIG. 11 is a control flow diagram showing control of the position-corresponding-emotion-evaluation device according to the fourth embodiment of the present teaching.
[FIG. 12] FIG. 12 is a schematic view of an overall configuration of a position-corresponding-emotion-evaluation device according to another embodiment of the present teaching.

### DESCRIPTION OF EMBODIMENT

Embodiments will be described hereinafter with reference to the drawings. In the drawings, the same or corresponding parts are denoted by the same reference numerals, and description thereof will not be repeated. The dimensions of components in the drawings do not strictly represent actual dimensions of the components and dimensional proportions of the components, for example.

### [First Embodiment]

### <Overall Configuration of Position-Corresponding-Emotion-Evaluation Device>

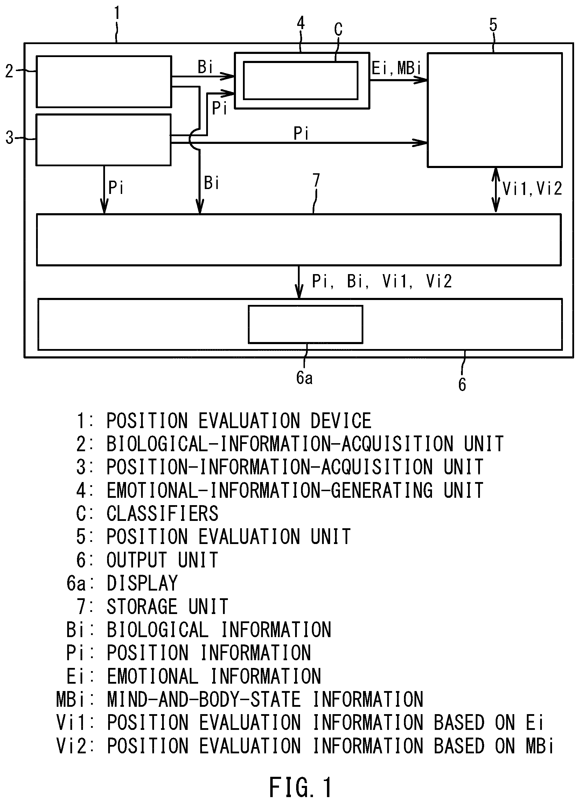

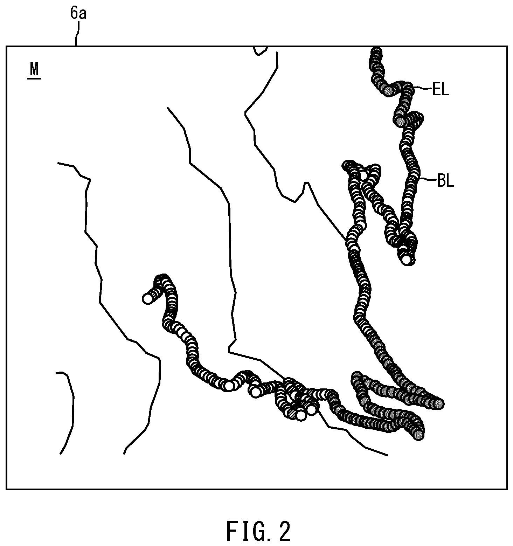

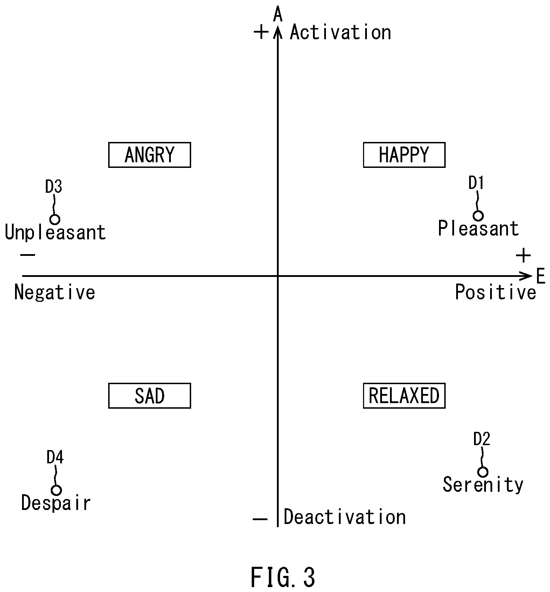

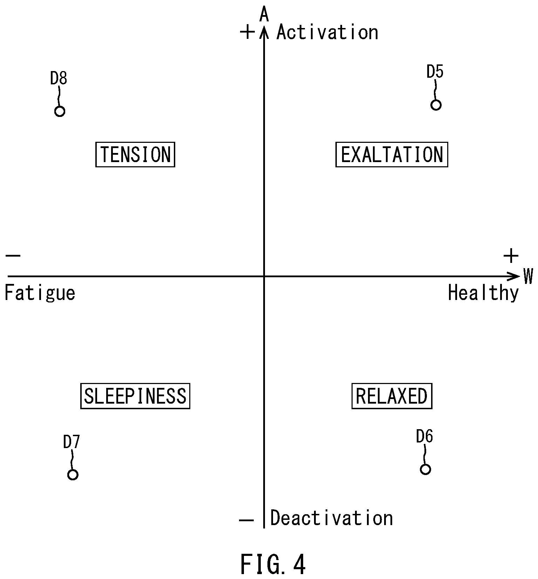

A position-corresponding-emotion-evaluation device 1 according to a first embodiment of the present teaching is now described with reference to FIG. 1. FIG. 1 is a schematic view of an overall configuration of the position-corresponding-emotion-evaluation device 1 according to the embodiment of the present teaching. FIG. 2 is a schematic view of a mode of display of position-corresponding-emotion-associated information Vi1 and position-corresponding-mind-and-body-state-associated information Vi2 by the position-corresponding-emotion-evaluation device 1 according to the first embodiment of the present teaching. FIG. 3 is a graph showing classification indexes of emotional information Ei in the position-corresponding-emotion-evaluation device 1 according to the first embodiment of the present teaching. FIG. 4 is a graph showing classification indexes of mind-and-body-state information MBi in the position-corresponding-emotion-evaluation device 1 according to the first embodiment of the present teaching.

As shown in FIG. 1, the position-corresponding-emotion-evaluation device 1 according to the first embodiment of the present teaching is a device for evaluating position information Pi on a place where a target person is located, based on emotional information Ei on an emotion of the target person. The position-corresponding-emotion-evaluation device 1 generates emotional information Ei on an emotion and mind-and-body-state information MBi on a mind and body state, based on biological information Bi on at least a heartbeat of the target person. The position-corresponding-emotion-evaluation device 1 generates, based on position information Pi and the generated emotional information Ei, position-corresponding-emotion-associated information Vi1 that includes the emotional information Ei associated with the position information Pi, or the position information Pi associated with the emotional information Ei. Furthermore, the position-corresponding-emotion-evaluation device 1 generates, based on the position information Pi and the generated mind-and-body-state information MBi, position-corresponding-mind-and-body-state-associated information Vi2 that includes the mind-and-body-state information MBi associated with the position information Pi, or the position information Pi associated with the mind-and-body-state information MBi.

The position-corresponding-emotion-evaluation device 1 includes a biological-information-acquisition unit 2, a position-information-acquisition unit 3, an emotional-information-generating unit 4, a position evaluation unit 5, an output unit 6, and a storage unit 7.

The biological-information-acquisition unit 2 measures biological information Bi of a target person, and acquires the measured biological information Bi. The biological-information-acquisition unit 2 stores the acquired biological information Bi on the storage unit 7. The biological-information-acquisition unit 2 includes a variety of sensors for acquiring biological information Bi. The biological-information-acquisition unit 2 measures, per unit of time, biological information Bi including, for example, body surface temperature, triaxial acceleration, information on a heartbeat that is a heart rate, a heartbeat waveform, a cardiac cycle, a change in heart rate, blood pressure, a high-frequency fluctuation component (HF) and a low-frequency fluctuation component (LF), regarding the target person, and its acquisition time. The biological-information-acquisition unit 2 includes, for example, a temperature sensor, an acceleration sensor and a heart rate sensor.

The position-information-acquisition unit 3 acquires position information Pi on a place where the target person is located (hereinafter referred to simply as "position information Pi"). The position-information-acquisition unit 3 stores the acquired position information Pi on the storage unit 7. The position-information-acquisition unit 3 has, for example, a global navigation satellite system (GNSS) receiver for acquiring position information Pi. The GNSS receiver is a receiver constituting a GNSS. The GNSS receiver receives a distance measuring radio wave from a satellite, and calculates latitude, longitude and altitude that are absolute coordinates of the GNSS receiver. The position-information-acquisition unit 3 acquires, per unit of time, position information Pi that includes, for example, the latitude, longitude, altitude and direction of the place where the target person is located, and its acquisition time.

The emotional-information-generating unit 4 is a part of a control unit for generating emotional information Ei on an emotion of the target person (hereinafter referred to simply as "emotional information Ei") and mind-and-body-state information MBi on a mind and body state of the target person (hereinafter referred to simply as "mind-and-body-state information MBi), based on biological information Bi of the target person. The emotional-information-generating unit 4 has, in advance, information on the target person, such as gender, age and physical features of the target person. The emotional-information-generating unit 4 has at least one classifier C that is a criterion for generating the emotional information Ei on an emotion and the mind-and-body-state information MBi based on the biological information Bi on at least a heartbeat of the target person. In this embodiment, the emotional-information-generating unit 4 includes classifiers C of different types.

The emotional-information-generating unit 4 acquires biological information Bi on at least a heartbeat of the target person from the biological-information-acquisition unit 2. The emotional-information-generating unit 4 determines a classifier C to be used, based on information on the target person, or in accordance with selection by the target person. In addition, the emotional-information-generating unit 4 can determine a means of transportation of the target person (for example, a motorcycle, a bicycle, and on foot) based on time included in position information Pi acquired by the position-information-acquisition unit 3. The emotional-information-generating unit 4 determines a classifier C in accordance with the means of transportation. In addition, the emotional-information-generating unit 4 can determine whether the target person is riding on a leaning vehicle, based on the triaxial acceleration of the target person acquired by the biological-information-acquisition unit 2. The emotional-information-generating unit 4 determines a classifier C in accordance with a vehicle type.

The emotional-information-generating unit 4 calculates, by using the determined classifier C, evaluation data D provided by at least one of parameter values that are, for example, an R-R interval, an LF/HF ratio and a frequency component in biological information Bi on a heartbeat of the target person. The classifier C makes a classification of the calculated evaluation data D. The classifier C classifies each piece of evaluation data D calculated per unit of time, by using a pattern recognition model such as a support vector machine (SVM). The classifier C assigns emotional information Ei and mind-and-body-state information MBi to the evaluation data D classified by the pattern recognition model.

The position evaluation unit 5 is a part of the control unit for generating position-corresponding-emotion-associated information Vi1 in which position information Pi and emotional information Ei are associated, as well as position-corresponding-mind-and-body-state-associated information Vi2 in which position information Pi and mind-and-body-state information MBi are associated. The position evaluation unit 5 has map information M. The position evaluation unit 5 acquires position information Pi from the position-information-acquisition unit 3. The position evaluation unit 5 also acquires emotional information Ei and mind-and-body-state information MBi from the emotional-information-generating unit 4.

The position evaluation unit 5 generates, per unit of time, position-corresponding-emotion-associated information Vi1 in which position information Pi and emotional information Ei are associated by using time as a flag, for example. Likewise, the position evaluation unit 5 generates, per unit of time, position-corresponding-mind-and-body-state-associated information Vi2 in which position information Pi and mind-and-body-state information MBi are associated, by using time as a flag, for example. That is, the position evaluation unit 5 acquires emotional information Ei and mind-and-body-state information MBi generated based on biological information Bi on at least a heartbeat of the target person, as well as position information Pi when the biological information Bi has been acquired from the target person, and then associates the place where the target person is located with the emotion and the mind and body state of the target person. Thus, the position evaluation unit 5 can evaluate the place where the target person is located, by the emotion and the mind and body state of the target person.

The position evaluation unit 5 can output, to the output unit 6, emotional information Ei associated with position information Pi, or position information Pi associated with emotional information Ei, included in position-corresponding-emotion-associated information Vi1. The position evaluation unit 5 can output, to the output unit 6, mind-and-body-state information MBi associated with position information Pi, or position information Pi associated with mind-and-body-state information MBi, included in position-corresponding-mind-and-body-state-associated information Vi2.

The output unit 6 is a part of the control unit for outputting information included in position-corresponding-emotion-associated information Vi1 and position-corresponding-mind-and-body-state-associated information Vi2. The output unit 6 acquires biological information Bi on at least a heartbeat of the target person from the biological-information-acquisition unit 2. The output unit also acquires position-corresponding-emotion-associated information Vi1 and position-corresponding-mind-and-body-state-associated information Vi2 from the position evaluation unit 5. The output unit 6 acquires emotional information Ei associated with position information Pi, or position information Pi associated with emotional information Ei, from the position evaluation unit 5. The output unit 6 further acquires mind-and-body-state information MBi associated with position information Pi, or position information Pi associated with mind-and-body-state information MBi, from the position evaluation unit 5. The output unit 6 outputs each piece of the acquired information to external devices, such as a display, a portable terminal, and a server. In addition, the output unit 6 may include a display 6a for displaying each piece of the information.

As shown in FIG. 2, the output unit 6 displays map information M on the display 6a based on position information Pi, for example. Furthermore, the output unit 6 displays, at a place where the target person is located on the map information M, an emotion label EL obtained by converting emotional information Ei (see FIG. 1) into at least one of a numerical value, a mark, a figure or a color, based on position-corresponding-emotion-associated information Vi1 (see FIG. 1). The output unit 6 displays a mind and body state label BL obtained by converting mind-and-body-state information MBi (see FIG. 1) into at least one of a numerical value, a mark, a figure, or a color, based on position-corresponding-mind-and-body-state-associated information Vi2 (see FIG. 1). In this embodiment, the output unit 6 displays, per unit of time, the emotion label EL and the mind and body state label BL by using a figure of a round shape in a manner to overlap the map information M.

The storage unit 7 stores, for example, biological information Bi detected at least by a wearable sensor that is the biological-information-acquisition unit 2, position information Pi detected by the GNSS receiver that is the position-information-acquisition unit 3, position-corresponding-emotion-associated information Vi1, and position-corresponding-mind-and-body-state-associated information Vi2. The storage unit 7 has, for example, memory inside a RAM and a processor, and a hard disk, for storing biological information Bi. The storage unit 7 stores, in time series, position-corresponding-emotion-associated information Vi1 and position-corresponding-mind-and-body-state-associated information Vi2 generated by the position evaluation unit 5. The storage unit 7 transmits, to the position evaluation unit 5 or the output unit 6, emotional information Ei associated with position information Pi, or position information Pi associated with emotional information Ei, included in the stored position-corresponding-emotion-associated information Vi1. The storage unit 7 transmits, to the position evaluation unit 5 or the output unit 6, mind-and-body-state information MBi associated with position information Pi, or position information Pi associated with mind-and-body-state information MBi, included in the position-corresponding-mind-and-body-state-associated information Vi2.

The biological-information-acquisition unit 2, the position-information-acquisition unit 3, the emotional-information-generating unit 4, the position evaluation unit 5, and the output unit 6 that are parts of the control unit may be practically configured such that a CPU, a ROM, a RAM and an HDD, for example, are connected via a bus. The biological-information-acquisition unit 2, the position-information-acquisition unit 3, the emotional-information-generating unit 4, and the position evaluation unit 5 may be configured as, for example, a one-chip LSI. The biological-information-acquisition unit 2 and the position-information-acquisition unit 3 store various programs and data for acquisition and storage of respective pieces of information. The emotional-information-generating unit 4 stores various programs and data for controlling the evaluators. The position evaluation unit 5 stores various programs and data for acquiring, and associating, position information Pi, emotional information Ei and mind-and-body-state information MBi.

### [Configuration of Classifier C]

The classifiers C of the emotional-information-generating unit 4 are now described with reference to FIGS. 3 and 4.

As shown in FIG. 3, a classifier C of the emotional-information-generating unit 4 classifies evaluation data D, for example, with a valence value E and an arousal value A as indexes, by using a pattern recognition model. The classifier C acquires a valence value E and an arousal value A of the classified evaluation data D. The classifier C generates emotional information Ei by selecting an emotion of a target person from a plurality of types of emotions, based on the valence value E and the arousal value A.

A valence value E represents the degree ranging from a positive emotion to a negative emotion. The positive emotion includes favorable emotions of the target person, such as excitement, ease, exaltation, serenity, calm, and pleasantness. The negative emotion includes unfavorable emotions of the target person, such as tension, agitation, depression, anxiety, unpleasantness, and despair. A positive emotion is assigned to evaluation data D having a high valence value E (a positive (+) valence value E) in accordance with the valence value E. A negative emotion is assigned to evaluation data D having a low valence value E (a negative (-) valence value E) in accordance with the valence value E.

An arousal value A represents the degree ranging from an activated state of emotion to a deactivated state of emotion. An activated emotion includes a heightened state of emotion of the target person, such as excitement, exaltation, pleasantness, agitation, tension, and unpleasantness. A deactivated emotion includes a lowered state of emotion of the target person, such as ease, serenity, calm, depression, anxiety, and despair. An activated emotion is assigned to evaluation data D having a high arousal value A (a positive (+) arousal value A) in accordance with the arousal value A. A deactivated emotion is assigned to evaluation data D having a low arousal value A (a negative (-) arousal value A) in accordance with the arousal value A.

The classifier C assigns an emotion represented by evaluation data D, based on the valence data E and the arousal data A of the evaluation data D classified in accordance with the pattern recognition model. In a case where the valence value E of the evaluation data D falls within a range of positive emotions (a positive (+) valence value E), and the arousal value A of the evaluation data D falls within a range of activated emotions (a positive (+) arousal value A), the classifier C assigns the evaluation data D to a region of "Happy" that includes emotions, such as excitement, exaltation, and pleasantness. Furthermore, the classifier C assigns a specific emotion (for example, excitement, exaltation and pleasantness) to the evaluation data D within the region of "Happy", based on the valence value E and the arousal value A. For example, in a case where evaluation data D1 has a high valence value E (i.e., a high degree of positive emotion), while having a low arousal value A (i.e., a low degree of activated emotion), in the region of "Happy", the classifier C selects the emotion of "Pleasant" for the evaluation data D1 from a plurality of types of emotions in the region of "Happy".

In a case where a valence value E of evaluation data D falls within the range of positive emotions (a positive (+) valence value E), and an arousal value A of the evaluation data D falls within a range of deactivated emotions (a negative (-) arousal value A), the classifier C assigns the evaluation data D to a region of "Relaxed" that includes emotions, such as ease, serenity and calm. Furthermore, the classifier C assigns a specific emotion (for example, ease, serenity and calm) to the evaluation data D within the region of "Relaxed", based on the valence value E and the arousal value A. For example, in a case where evaluation data D2 has a high valence value E (i.e., a high degree of positive emotion), while having a low arousal value A (i.e., a high degree of deactivated emotion), in the region of "Relaxed", the classifier C selects the emotion of "Serenity" for the evaluation data D2 from a plurality of types of emotions in the region of "Relaxed".

In a case where a valence value E of evaluation data D falls within a range of negative emotions (a negative (-) valence value E), and an arousal value A of the evaluation data D falls within the range of activated emotions (a positive (+) arousal value A), the classifier C assigns the evaluation data D to a region of "Angry" that includes emotions, such as tension, agitation, and unpleasantness. Furthermore, the classifier C assigns a specific emotion (for example, tension, agitation, and unpleasantness) to the evaluation data D within the region of "Angry", based on the valence value E and the arousal value A. For example, in a case where evaluation data D3 has a low valence value E (i.e., a high degree of negative emotion), while having a low arousal value A (i.e., a low degree of activated emotion), in the region of "Angry", the classifier C selects the emotion of "Unpleasant" for the evaluation data D3 from a plurality of types of emotions in the region of "Angry".

In a case where a valence value E of evaluation data D falls within the range of negative emotions (a negative (-) valence value E), and an arousal value A of the evaluation data D falls within the range of deactivated emotions (a negative (-) arousal value A), the classifier C assigns the evaluation data D to a region of "Sad" that includes emotions, such as depression, anxiety, and despair. Furthermore, the classifier C assigns a specific emotion (for example, depression, anxiety, and despair) to the evaluation data D within the region of "Sad", based on the valence value E and the arousal value A. For example, in a case where evaluation data D4 has a low valence value E (i.e., a high degree of negative emotion), while having a low arousal value A (i.e., a high degree of deactivated emotion), in the region of "Sad", the classifier C selects the emotion of "Despair" for the evaluation data D4 from a plurality of types of emotions in the region of "Sad".

Furthermore, as shown in FIG. 4, a classifier C classifies the evaluation data D, for example, with a willpower value W and an arousal value A as indexes, by using a pattern recognition model. The classifier C acquires a willpower value W and an arousal value A of the classified evaluation data D. The classifier C generates mind-and-body-state information MBi on a mind and body state of a target person based on a plurality of types of mind and body states, based on the willpower value W and the arousal value A.

A willpower value W represents the degree ranging from a healthy mind and body state to a fatigued mind and body state. A mind and body state including, for example, excitement and relaxedness is assigned to evaluation data D having a high willpower value W (a positive (+) willpower value W) in accordance with the willpower value W. A mind and body state including, for example, tension and sleepiness is assigned to evaluation data D having a low willpower value W (a negative (-) willpower value W) in accordance with the willpower value W.

An arousal value A represents the degree ranging from an activated state of emotion to a deactivated state of emotion. A mind and body state including, for example, exaltation and tension is assigned to evaluation data D having a high arousal value A (a positive (+) arousal value A) in accordance with the arousal value A. A mind and body state including, for example, relaxedness and sleepiness is assigned to evaluation data D having a low arousal value A (a negative (-) arousal value A) in accordance with the arousal value A.

In a case where a willpower value W of evaluation data D5 falls within a range of healthy states (a positive (+) willpower value W), and an arousal value A of the evaluation data D5 falls within a range of activated emotions (a positive (+) arousal value A), the classifier C assigns the evaluation data D5 to a region of mind and body state of "Exaltation".

In a case where a willpower value W of evaluation data D6 falls within the range of healthy states (a positive (+) willpower value W), and an arousal value A of the evaluation data D6 falls within a range of deactivated emotions (a negative (-) arousal value A), the classifier C assigns the evaluation data D6 to a region of mind and body state of "Relaxed".

In a case where a willpower value W of evaluation data D7 falls within a range of fatigued states (a negative (-) willpower value W), and an arousal value A of the evaluation data D7 falls within the range of deactivated emotions (a negative (-) arousal value A), the classifier C assigns the evaluation data D7 to a region of mind and body state of "Sleepiness".

In a case where a willpower value W of evaluation data D8 falls within the range of fatigued states (a negative (-) willpower value W), and an arousal value A of the evaluation data D8 falls within the range of activated emotions (a positive (-) arousal value A), the classifier C assigns the evaluation data D8 to a region of mind and body state of "Tension".

The classifier C thus configured classifies evaluation data D by using a pattern recognition model such as a support vector machine (SVM). The pattern recognition model is generated by using, as training data, emotional information Ei on an emotion and biological information Bi on at least a heartbeat, of each test subject under the influence of a variety of external events. The training data includes numerous pairs of input information provided by a parameter value, such as an R-R interval, an LF/HF ratio and a frequency component that are calculated based on biological information on a heartbeat of each test subject when seeing a particular image, for example, and self-assessed emotional information by the test subject seeing the particular image, where the pairs of input information and self-assessed emotional information are labeled with correct answers. The classifiers C enhances its classification accuracy through machine learning using training data.

The classifiers C provide different classification accuracy in accordance with constituents of training data. For example, in a case where a classifier C uses training data including the pairs obtained from male test subjects only, the classification accuracy of emotions of male target persons enhances. In a case where a classifier C uses training data including the pairs obtained from test subjects in their thirties and forties only, the classification accuracy of emotions of target persons in their thirties and forties enhances. In this manner, classifiers C of different types are provided through the use of learning data classified based on information on test subjects.

As shown in FIG. 1, the emotional-information-generating unit 4 is connected to the biological-information-acquisition unit 2. The emotional-information-generating unit 4 can acquire biological information Bi on at least a heartbeat of a target person from the biological-information-acquisition unit 2. The emotional-information-generating unit 4 is also connected to the position-information-acquisition unit 3. The emotional-information-generating unit 4 can acquire position information Pi on a place where the target person is located, from the position-information-acquisition unit 3.

The emotional-information-generating unit 4 is connected to the position evaluation unit 5. The emotional-information-generating unit 4 can transmit, to the position evaluation unit 5, emotional information Ei on an emotion of the target person, as well as position information Pi on a place where the target person is located.

The emotional-information-generating unit 4 is connected to the output unit 6. The emotional-information-generating unit 4 can transmit, to the output unit 6, at least one of biological information Bi on a heartbeat, position information Pi, emotional information Ei, or mind-and-body-state information MBi, of the target person.

The position evaluation unit 5 is connected to the output unit 6. The position evaluation unit 5 can transmit, to the output unit 6, map information M, emotional information Ei associated with position information Pi, or position information Pi associated with emotional information Ei, included in position-corresponding-emotion-associated information Vi1, and mind-and-body-state information MBi associated with position information Pi, or position information Pi associated with mind-and-body-state information MBi, included in position-corresponding-mind-and-body-state-associated information Vi2.

### <Control Flow>

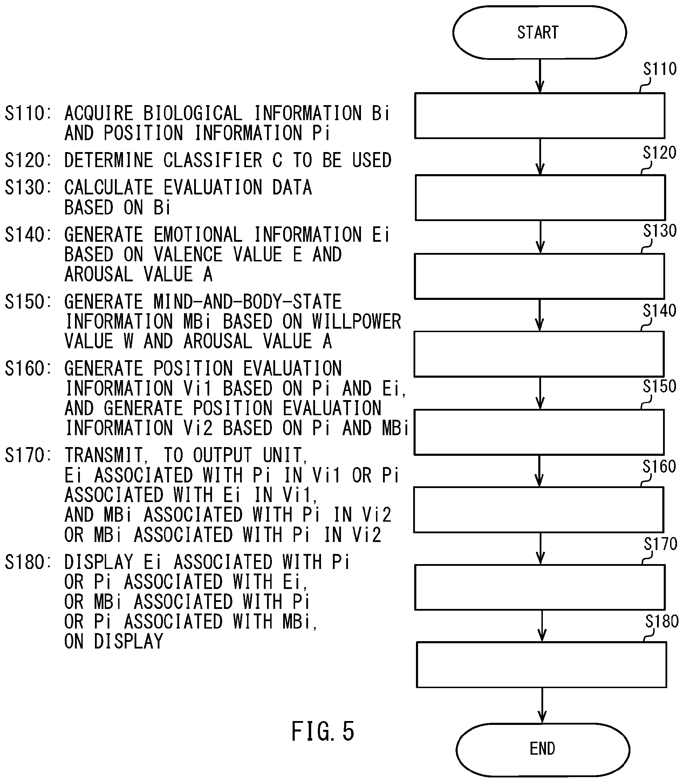

A control flow of control of position-corresponding emotion evaluation by the position-corresponding-emotion-evaluation device 1 is now described with reference to FIG. 5. FIG. 5 is a control flow diagram showing control of the position-corresponding-emotion-evaluation device 1 according to the first embodiment of the present teaching. It is to be noted that the position-corresponding-emotion-evaluation device 1 has already acquired a start signal for position-corresponding emotion evaluation.

As shown in FIG. 5, the position-corresponding-emotion-evaluation device 1 acquires, per unit of time, biological information Bi on at least a heartbeat of a target person that includes time, for example, by the biological-information-acquisition unit 2, as well as position information Pi, at step S110.

At step S120, the emotional-information-generating unit 4 of the position-corresponding-emotion-evaluation device 1 determines classifiers C to be used, based on information on the target person or position information Pi, or in accordance with selection by the target person.

At step S130, the emotional-information-generating unit 4 calculates, per unit of time, evaluation data D from biological information Bi on at least a heartbeat.

At step S140, the emotional-information-generating unit 4 classifies evaluation data D calculated per unit of time, with a valence value E and an arousal value A as indexes, by a classifier C. The emotional-information-generating unit 4 generates emotional information Ei based on the valence value E and the arousal value A.

At step S150, the emotional-information-generating unit 4 classifies evaluation data D calculated per unit of time, with a willpower value W and an arousal value A as indexes, by a classifier C,. The emotional-information-generating unit 4 generates mind-and-body-state information MBi based on the willpower value W and the arousal value A.

At step S160, the position evaluation unit 5 of the position-corresponding-emotion-evaluation device 1 generates position-corresponding-emotion-associated information Vi1 per unit of time in which position information Pi and emotional information Ei are associated, and position-corresponding-mind-and-body-state-associated information Vi2 per unit of time in which position information Pi and mind-and-body-state information MBi are associated, by using time as a flag, for example.

At step S170, the position evaluation unit 5 transmits, to the output unit 6, emotional information Ei associated with position information Pi, or position information Pi associated with emotional information Ei, included in position-corresponding-emotion-associated information Vi1. The position evaluation unit 5 also transmits, to the output unit 6, mind-and-body-state information MBi associated with position information Pi, or position information Pi associated with mind-and-body-state information MBi, included in position-corresponding-mind-and-body-state-associated information Vi2.

At step S 180, the output unit 6 displays acquired emotional information Ei associated with position information Pi, or acquired position information Pi associated with emotional information Ei, on the display 6a. Alternatively, the output unit 6 displays mind-and-body-state information MBi associated with position information Pi, or position information Pi associated with mind-and-body-state information MBi, on the display 6a.

In the above-described configuration, the position-corresponding-emotion-evaluation device 1 generates, by using the classifiers C, emotional information Ei and mind-and-body-state information MBi of a target person, based on biological information Bi on at least a heartbeat of the target person that includes no external events, so that the device can reduce the processing load of hardware. Furthermore, the position-corresponding-emotion-evaluation device 1 associates emotional information Ei and mind-and-body-state information MBi generated by the emotional-information-generating unit 4, with position information Pi that can be easily acquired through the use of existing technologies and facilities, so that the device can add external events to the emotional information Ei and the mind-and-body-state information MBi without increasing the processing load of the hardware. This allows the position-corresponding-emotion-evaluation device 1 to convert the position information Pi merely indicating a place where the target person is located into value-added position information Pi indicating what the place means for the target person. Furthermore, the position-corresponding-emotion-evaluation device 1 adds position information Pi to emotional information Ei and mind-and-body-state information MBi, to thereby increase applications of the emotional information Ei and the mind-and-body-state information MBi. That is, the position-corresponding-emotion-evaluation device 1 can convert the emotional information Ei merely indicating an emotion of the target person and the mind-and-body-state information MBi merely indicating a mind and body state of the target person, into value-added emotional information Ei and mind-and-body-state information MBi that indicate what external events, such as a place, time, weather and season, caused the emotion and the mind and body state of the target person.

The position-corresponding-emotion-evaluation device 1 generates a plurality of types of emotional information Ei and a plurality of types of mind-and-body-state information MBi based on biological information Bi on at least a heartbeat of a target person, by the emotional-information-generating unit 4. The position-corresponding-emotion-evaluation device 1 generates emotional information Ei representing one emotion selected from a plurality of emotions and mind-and-body-state information MBi representing one mind and body state selected from a plurality of mind and body states for position information Pi, so that the device can flexibly respond to a change in emotion as well as mind and body state of the target person. The position-corresponding-emotion-evaluation device 1 also selects degree of an emotion, and is thereby capable of flexibly responding to a change in emotion of the target person.

The position-corresponding-emotion-evaluation device 1 can alert or advise the target person in a case where emotional information Ei representing a negative emotion is continuously generated, or emotional information Ei represents a high degree of negative emotion. Likewise, the position-corresponding-emotion-evaluation device 1 can alert or advise the target person in a case where mind-and-body-state information MBi representing a fatigued mind and body state is continuously generated, or mind-and-body-state information MBi represents a high degree of fatigued mind and body state. Thus, the position-corresponding-emotion-evaluation device 1 can effectively use information on an emotion, while suppressing an increase in the processing load of hardware.

### [Second Embodiment]

### < Position-Corresponding-Emotion-Evaluation Device 1 Used by One Person>

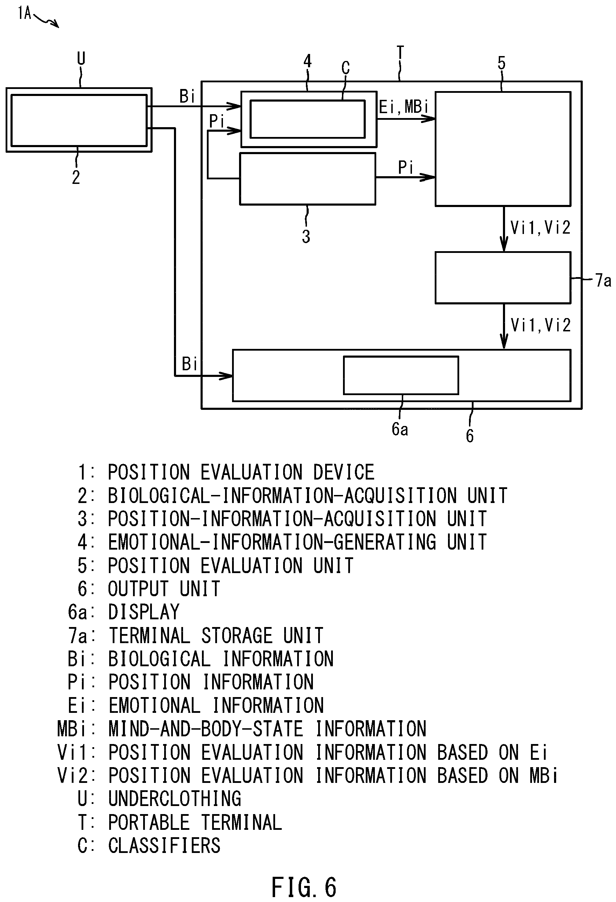

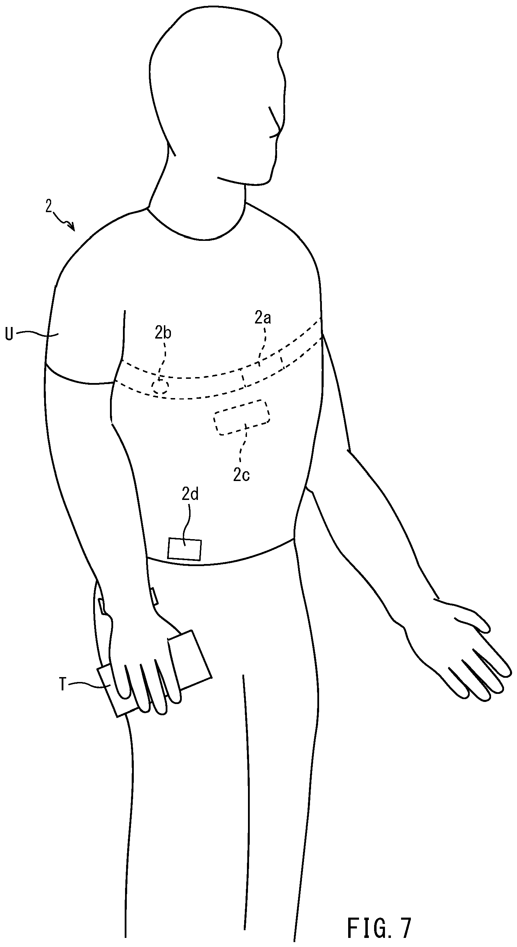

A position-corresponding-emotion-evaluation device 1A according to a second embodiment of the present teaching is now described with reference to FIGS. 6 and 7. FIG. 6 is a schematic view of an overall configuration of the position-corresponding-emotion-evaluation device 1A according to the second embodiment of the present teaching. FIG. 7 is a schematic view of a configuration of the position-corresponding-emotion-evaluation device 1A according to the second embodiment of the present teaching, which is worn by a target person. Note that, in the following embodiment, specific description of similar points to those in the embodiment already described will be omitted, and only a portion which differs from the already described embodiment will be described in detail. In this embodiment, the position-corresponding-emotion-evaluation device 1A is used solely by one target person.

As shown in FIG. 6, the position-corresponding-emotion-evaluation device 1A includes the biological-information-acquisition unit 2, the position-information-acquisition unit 3, the emotional-information-generating unit 4, the position evaluation unit 5, the output unit 6, and a terminal storage unit 7a.

As shown FIG. 7, the biological-information-acquisition unit 2 is a wearable sensor having a variety of sensors incorporated in, for example, a wearable device on the body of a target person, or clothing of the target person. In this embodiment, the biological-information-acquisition unit 2 is a wearable sensor with a heart rate sensor 2a, a temperature sensor 2b, a triaxial acceleration sensor 2c, communication equipment 2d, and a battery incorporated in underclothing U. The wearable sensor is configured such that, when it is worn by the target person, electrodes of the heart rate sensor 2a and a measuring terminal of the temperature sensor 2b make contact with the chest of the target person. The wearable sensor, serving as the biological-information-acquisition unit 2, can transmit biological information Bi detected by the heart rate sensor 2a, the temperature sensor 2b, and the triaxial acceleration sensor 2c to the terminal storage unit 7a, as well as receive, from the control unit, control signals to be transmitted to the heart rate sensor 2a, the temperature sensor 2b, and the triaxial acceleration sensor 2c, by using the communication equipment 2d.

The position-information-acquisition unit 3, the emotional-information-generating unit 4, the position evaluation unit 5, and the output unit 6 are incorporated in a portable terminal T such as a smartphone carried by the target person. The position-information-acquisition unit 3 is constituted of a GNSS receiver incorporated in the portable terminal T. The position-information-acquisition unit 3 can transmit acquired position information Pi to the terminal storage unit 7a. The emotional-information-generating unit 4, the position evaluation unit 5, and the output unit 6 that are parts of the control unit are stored in a ROM of the portable terminal T, for example. The emotional-information-generating unit 4, the position evaluation unit 5, and the output unit 6 are controllable by a CPU of the portable terminal T. The portable terminal T can communicate with the wearable sensor and the GNSS receiver via communication equipment. Accordingly, the emotional-information-generating unit 4 and the output unit 6 can communicate with the biological-information-acquisition unit 2 and the position-information-acquisition unit 3 via the communication equipment of the portable terminal T.

As shown in FIG. 6, the terminal storage unit 7a serving as a storage part stores at least biological information Bi detected by the wearable sensor serving as the biological-information-acquisition unit 2, position information Pi detected by the GNSS receiver serving as the position-information-acquisition unit 3, position-corresponding-emotion-associated information Vi1, and position-corresponding-mind-and-body-state-associated information Vi2. The terminal storage unit 7a is constituted of a storage device of the portable terminal T (see FIG. 7). The terminal storage unit 7a stores, in time series, position-corresponding-emotion-associated information Vi1 and position-corresponding-mind-and-body-state-associated information Vi2 that are generated by the position evaluation unit 5. The terminal storage unit 7a transits, to the output unit 6, emotional information Ei associated with position information Pi, or position information Pi associated with emotional information Ei, included in stored position-corresponding-emotion-associated information Vi1. The position evaluation unit 5 can transmit, to the output unit 6, mind-and-body-state information MBi associated with position information Pi, or position information Pi associated with mind-and-body-state information MBi included in position-corresponding-mind-and-body-state-associated information Vi2.

The position-corresponding-emotion-evaluation device 1A retrieves position-corresponding-emotion-associated information Vi1 and position-corresponding-mind-and-body-state-associated information Vi2, of the past, from the terminal storage unit 7a, so that the device can compare pieces of position-corresponding-emotion-associated information Vi1 obtained at the same position on different dates, as well as pieces of position-corresponding-mind-and-body-state-associated information Vi2 obtained at the same position on different dates.

The output unit 6 includes the display 6a of the portable terminal T (see FIG. 7). The output unit 6 is connected to the terminal storage unit 7a. The output unit 6 can acquire position-corresponding-emotion-associated information Vi1 from the terminal storage unit 7a. The output unit 6 is configured to be capable of acquiring at least one of biological information Bi, position information Pi, emotional information Ei, or mind-and-body-state information MBi, from the terminal storage unit 7a. The output unit 6 can display, on the display 6a of the portable terminal T, emotional information Ei associated with position information Pi, or position information Pi associated with emotional information Ei, included in position-corresponding-emotion-associated information Vi1. The output unit 6 can display, on the display 6a of the portable terminal T, mind-and-body-state information MBi associated with position information Pi, or position information Pi associated with mind-and-body-state information MBi, included in position-corresponding-mind-and-body-state-associated information Vi2. The output unit 6 can display at least one of biological information Bi, position information Pi, emotional information Ei, or mind-and-body-state information MBi. The output unit 6 can output at least one of biological information Bi on at least a heartbeat of the target person, position information Pi, emotional information Ei, mind-and-body-state information MBi, position-corresponding-emotion-associated information Vi1 based on emotional information Ei, or position-corresponding-mind-and-body-state-associated information Vi2 based on mind-and-body-state information MBi, to an external device via the communication equipment of the portable terminal T.

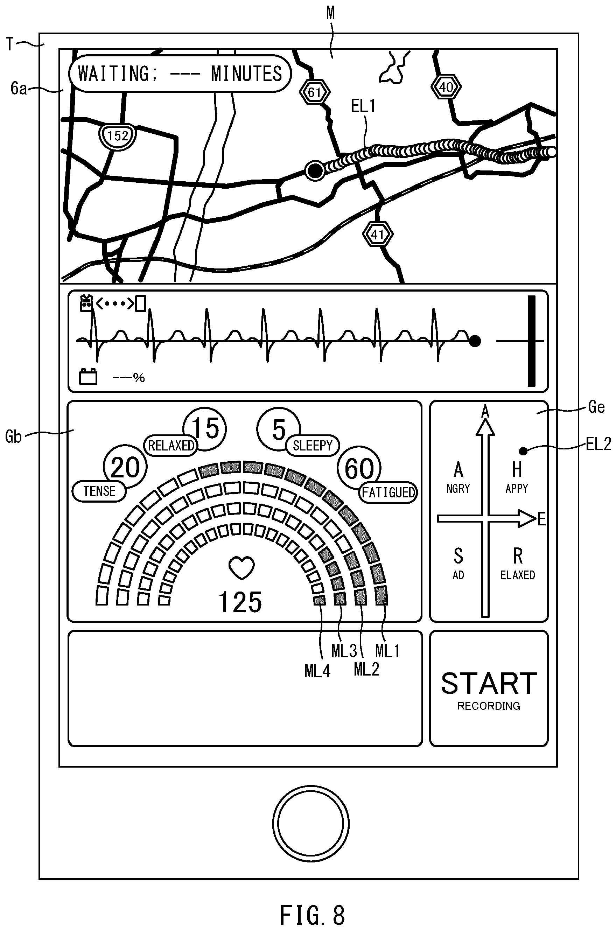

With reference to FIG. 8, a state of display of position-corresponding-emotion-associated information Vi1 and position-corresponding-mind-and-body-state-associated information Vi2 by the position-corresponding-emotion-evaluation device 1A is now described. FIG. 8 is a schematic view of the display state of the position-corresponding-emotion-associated information Vi1 and the position-corresponding-mind-and-body-state-associated information Vi2 in the position-corresponding-emotion-evaluation device 1A according to the second embodiment of the present teaching.

As shown in FIG. 8, the output unit 6 (see FIG. 6), for example, displays on the display 6a of the portable terminal T, map information M of a predetermined range centering around a place where a target person is located. The output unit 6 also displays position information Pi indicating the place where the target person is located on the map information M. At that time, the output unit 6 displays on the map, emotional information Ei associated with position information Pi included in position-corresponding-emotion-associated information Vi1, or mind-and-body-state information MBi associated with position information Pi included in position-corresponding-mind-and-body-state-associated information Vi2, in the form of an emotion label EL1 that is obtained by converting the emotional information Ei associated with the position information Pi or the mind-and-body-state information MBi associated with the position information Pi into at least one of a numerical value, a mark, a figure, or a color. The output unit 6 may display position information Pi associated with emotional information Ei included in position-corresponding-emotion-associated information Vi1, or position information Pi associated with mind-and-body-state information MBi. For example, the output unit 6 may statistically process pieces of position information Pi that include a place, environment, weather, season, time, or the like obtained when particular emotional information Ei or mind-and-body-state information MBi is generated, and display the position information Pi.

The output unit 6 displays emotional information Ei on an emotion graph Ge, for example. The output unit 6 displays the emotion graph Ge that indicates a valence value E on the horizontal axis and an arousal value A on the vertical axis perpendicular to the horizontal axis. The first quadrant of the emotion graph Ge with positive valence values E (positive emotions) and positive arousal values A (activated emotions) is defined as a region of "Happy". The second quadrant of the emotion graph Ge with positive valence values E (positive emotions) and negative arousal values A (deactivated emotions) is defined as a region of "Relaxed". The third quadrant of the emotion graph Ge with negative valence values E (negative emotions) and negative arousal values A (deactivated emotions) is defined as a region of "Sad". The fourth quadrant of the emotion graph Ge with negative valence values E (negative emotions) and positive arousal values A (activated emotions) is defined as a region of "Angry". The output unit 6 converts emotional information Ei into an emotion label EL2 represented by at least one of a numerical value, a mark, a figure, or a color, and displays the emotion label EL2 at a position corresponding to the current emotion of the target person on the emotion graph Ge.

The output unit 6 displays a graph of mind and body state Gb indicating mind-and-body-state information MBi on a mind and body state of the target person. The graph of mind and body state Gb displays a mind and body state label BL1 obtained by converting the degree of exaltation into at least one of a numerical value, a mark, a figure or a color, where exaltation is a mind and body state having a positive willpower value W and a positive arousal value A. Likewise, the graph of mind and body state Gb displays a mind and body state label BL2 indicating the degree of relaxedness that is a mind and body state having a positive willpower value W and a negative arousal value A, a mind and body state label BL3 indicating the degree of tension that is a mind and body state having a negative willpower value W and a positive arousal value A, and a mind and body state label BL4 indicating the degree of sleepiness that is a mind and body state having a negative willpower value W and a negative arousal value A. The output unit 6 displays, on the graph of mind and body state Gb, mind-and-body-state information MBi on a mind and body state of the target person based on the willpower value W and the arousal value A.

In the configuration described above, the position-corresponding-emotion-evaluation device 1A generates emotional information Ei of the target person and mind-and-body-state information MBi of the target person based on biological information Bi of the target person, through the use of, for example, the classifiers C operated by hardware of the portable terminal T carried by the target person. The position-corresponding-emotion-evaluation device 1A adds an external event to the emotional information Ei and the mind-and-body-state information MBi without an increase in the processing load of the hardware, by using position information Pi acquired by the GNSS receiver of the portable terminal T. The position-corresponding-emotion-evaluation device 1A also adds an emotion and a mind and body state of the target person to the position information Pi without an increase in the processing load of the hardware. This allows the position-corresponding-emotion-evaluation device 1A to effectively use information on an emotion, while suppressing an increase in the processing load of the hardware.

### [Third Embodiment]

### <Position-Corresponding-Emotion-Evaluati on System>

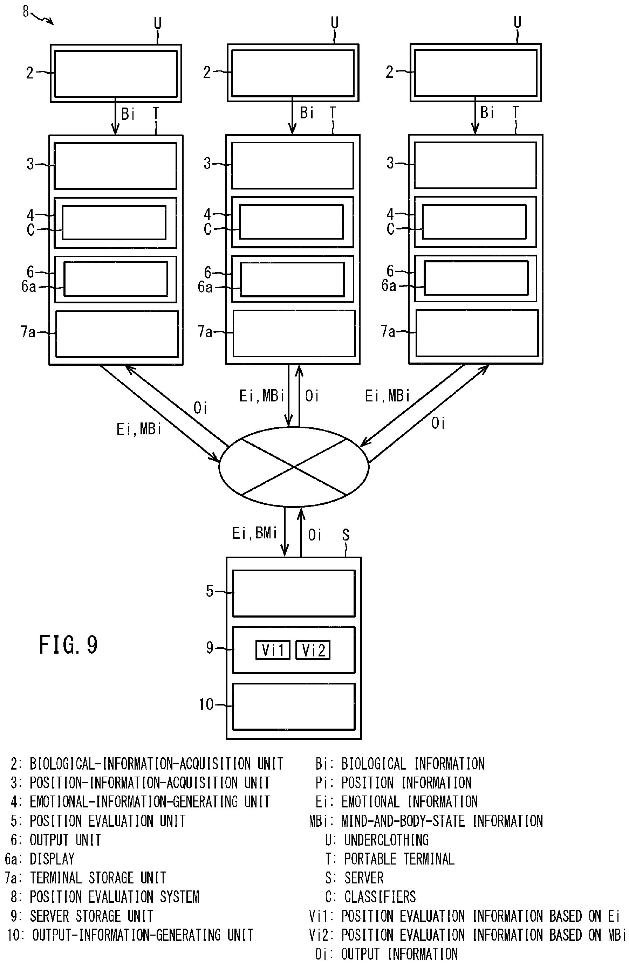

A position-corresponding-emotion-evaluation system 8 according to a third embodiment of the present teaching is now described with reference to FIG. 9. FIG. 9 is a schematic view of an overall configuration of the position-corresponding-emotion-evaluation system 8 according to the embodiment of the present teaching.

The position-corresponding-emotion-evaluation system 8 is a system for evaluating a position by using emotional information Ei and mind-and-body-state information MBi of a plurality of target persons. The position-corresponding-emotion-evaluation system 8 includes the biological-information-acquisition units 2, the position-information-acquisition units 3, the emotional-information-generating units 4, the position evaluation unit 5, the terminal storage units 7a, an output-information-generating unit 10, a server storage unit 9, and the output units 6. The position-corresponding-emotion-evaluation system 8 is constituted of the wearable sensors provided in underclothing U, for example, the portable terminals T and a server S.

Each of the biological-information-acquisition units 2 acquires biological information Bi. The biological-information-acquisition unit 2 is a wearable sensor having a variety of sensors incorporated in a wearable device on the body of a target person or in the underclothing U of the target person. That is, the biological-information-acquisition unit 2 is held by the target person (see FIG. 7). The biological-information-acquisition unit 2 can acquire biological information Bi of the target person wearing the underclothing U. The biological-information-acquisition unit 2 can transmit the biological information Bi of the target person to the server storage unit 9.

Each of the position-information-acquisition units 3 acquires position information Pi. The position-information-acquisition unit 3 is configured in each of the portable terminals T such as a smartphone carried by the target person (see FIG. 7). The position-information-acquisition unit 3 acquires position information Pi of the target person carrying the portable terminal T. The position-information-acquisition unit 3 can transmit the acquired position information Pi to the server storage unit 9.

Each of the emotional-information-generating units 4 that is a part of a control unit generates emotional information Ei and mind-and-body-state information MBi based on biological information Bi. The emotional-information-generating unit 4 is configured in the portable terminal T such as a smartphone carried by the target person. The portable terminal T is configured to be capable of communication via the communication equipment 2d of the wearable sensor. That is, the emotional-information-generating unit 4 is configured to be capable of communicating with the biological-information-acquisition unit 2 via the communication equipment of the portable terminal T. The emotional-information-generating unit 4 acquires biological information Bi from the underclothing U worn by the target person carrying the portable terminal T.

The portable terminal T includes the terminal storage unit 7a storing at least position-corresponding-emotion-associated information Vi1 and position-corresponding-mind-and-body-state-associated information Vi2. The terminal storage unit 7a is constituted of a storage device of the portable terminal T. The terminal storage unit 7a stores, in time series, position-corresponding-emotion-associated information Vi1 and position-corresponding-mind-and-body-state-associated information Vi2 that are generated by the position evaluation unit 5. The terminal storage unit 7a may store biological information Bi on a heartbeat, position information Pi, emotional information Ei and mind-and-body-state information MBi.

The position evaluation unit 5 that is a part of the control unit generates position-corresponding-emotion-associated information Vi1 and position-corresponding-mind-and-body-state-associated information Vi2. The position evaluation unit 5 is configured in the server S. The server S is connected to the Internet. The server S is configured to be capable of communicating with the portable terminal T carried by the target person via the Internet and the communication equipment of the portable terminal T. That is, the position evaluation unit 5 is configured to be capable of communicating with the position-information-acquisition unit 3 and the emotional-information-generating unit 4 via the Internet and the communication equipment of the portable terminal T.

The server storage unit 9, serving as a storage part, is constituted of a storage device of the server S. The server storage unit 9 stores at least position-corresponding-emotion-associated information Vi1 and position-corresponding-mind-and-body-state-associated information Vi2. The server storage unit 9 stores, in time series, position-corresponding-emotion-associated information Vi1 and position-corresponding-mind-and-body-state-associated information Vi2 that are generated by the position evaluation unit 5. The server storage unit 9 can transmit the stored position-corresponding-emotion-associated information Vi1 and the stored position-corresponding-mind-and-body-state-associated information Vi2 to the output-information-generating unit 10. The server storage unit 9 may store biological information Bi on a heartbeat, position information Pi, emotional information Ei, and mind-and-body-state information MBi.

The output-information-generating unit 10 generates output information Oi based on the stored position-corresponding-emotion-associated information Vi1 and the stored position-corresponding-mind-and-body-state-associated information Vi2. The output-information-generating unit 10 is configured in the server S. The output-information-generating unit 10 generates, as output information Oi, emotional information Pi associated with position information Pi, or position information Pi associated with emotional information Pi, or position information Pi associated with emotional information Pi, included in the position-corresponding-emotion-associated information Vi1. The output-information-generating unit 10 generates, as output information Oi, mind-and-body-state information MBi associated with position information Pi, or position information Pi associated with mind-and-body-state information MBi, included in the position-corresponding-mind-and-body-state-associated information Vi2. Furthermore, the output-information-generating unit 10 can generate output information Oi based on at least one of biological information Bi, position information Pi, emotional information Ei, or mind-and-body-state information MBi. The output-information-generating unit 10 can output the output information Oi to each of the output units 6 or an external device via the Internet and the communication equipment of the portable terminal T.

For example, the output-information-generating unit 10 generates output information Oi for displaying on the map information M the emotion label EL or the mind and body state label BL (see FIG. 8) that is obtained by converting at least one of emotional information Ei associated with position information Pi concerning an emotion of the target person at a place where the target person is located, or mind-and-body-state information MBi associated with position information Pi concerning a mind and body state of the target person at the place where the target person is located, into at least one of a numerical value, a mark, a figure, or a color.

The output unit 6 outputs output information Oi. The output unit 6 includes the display 6a of the portable terminal (see FIG. 8). The output unit 6 displays the output information Oi on the display 6a of the portable terminal T. The output unit 6 acquires the output information Oi from the output-information-generating unit 10 via the Internet and the communication equipment of the portable terminal T.

In the position-corresponding-emotion-evaluation system 8 thus configured, the server S is configured to be capable of acquiring pieces of position information Pi, pieces of emotional information Ei and pieces of mind-and-body-state information MBi from the portable terminals T carried by a plurality of target persons, via the Internet and the communication equipment of each of the portable terminals T.

The position-corresponding-emotion-evaluation system 8 stores, in the server storage unit 9, the pieces of position information Pi, the pieces of emotional information Ei and the pieces of mind-and-body-state information MBi acquired from the portable terminals T carried by the plurality of target persons. The position-corresponding-emotion-evaluation system 8 further stores, in the server storage unit 9, pieces of position-corresponding-emotion-associated information Vi1 and pieces of position-corresponding-mind-and-body-state-associated information Vi2 generated based on the pieces of position information Pi, the pieces of emotional information Ei, and the pieces of mind-and-body-state information MBi acquired from the respective portable terminals T.

The output-information-generating unit 10 of the position-corresponding-emotion-evaluation system 8 extracts emotional information Ei associated with position information, or position information Pi associated with emotional information Ei, from the position-corresponding-emotion-associated information Vi1 stored in the server storage unit 9. The output-information-generating unit 10 extracts mind-and-body-state information MBi associated with position information Pi, or position information Pi associated with mind-and-body-state information MBi, from the position-corresponding-mind-and-body-state-associated information Vi2. For example, the output-information-generating unit 10 extracts a plurality of pieces of position information Pi each associated with a piece of emotional information Ei at a particular place, and a plurality of pieces of position information Pi each associated with a piece of mind-and-body-state information MBi at the particular place. The output-information-generating unit 10 extracts, for example, a plurality of pieces of position information Pi each associated with a piece of emotional information Ei at a particular time (a particular season, a particular time frame, for example), and a plurality of pieces of position information Pi each associated with a piece of mind-and-body-state information MBi at the particular time.

The output-information-generating unit 10 generates output information Oi based on the extracted pieces of emotional information Ei each associated with a piece of position information Pi, or the extracted pieces of position information Pi each associated with a piece of emotional information Ei. The output-information-generating unit 10 generates output information Oi based on the extracted pieces of mind-and-body-state information MBi each associated with a piece of position information Pi, or the extracted pieces of position information Pi each associated with a piece of mind-and-body-state information MBi. The output-information-generating unit 10 generates, for example, output information Oi for displaying on the map information M respective pieces of information extracted from pieces of position-corresponding-emotion-associated information Vi1 and pieces of position-corresponding-mind-and-body-state-associated information Vi2 in a manner to overlap the map information M. Furthermore, the output-information-generating unit 10 generates, for example, output information Oi by processing respective pieces of information extracted from pieces of position-corresponding-emotion-associated information Vi1 and pieces of position-corresponding-mind-and-body-state-associated information Vi2 through the use of appropriate statistical methods.

The position-corresponding-emotion-evaluation system 8 thus configured generates a collection of position-corresponding-emotion-associated information Vi1 (big data) and a collection of position-corresponding-mind-and-body-state-associated information Vi2, respectively, by using a plurality of pieces of position-corresponding-emotion-associated information Vi1 generated from a plurality of pieces of emotional information Ei and a plurality of pieces of position information Pi, and a plurality of pieces of position-corresponding-mind-and-body-state-associated information Vi2 generated from a plurality of pieces of mind-and-body-state information MBi and a plurality of pieces of position information Pi. The position-corresponding-emotion-evaluation system 8 generates output information Oi based on the collection of position-corresponding-emotion-associated information Vi1 and the collection of position-corresponding-mind-and-body-state-associated information Vi2, based on arbitrary indexes. Accordingly, the position-corresponding-emotion-evaluation system 8 can exclude the influence of variation in pieces of position-corresponding-emotion-associated information Vi1 and pieces of position-corresponding-mind-and-body-state-associated information Vi2, due to differences in individuals' personality, such as character and taste of each individual. Furthermore, the position-corresponding-emotion-evaluation system 8 can evaluate pieces of position-corresponding-emotion-associated information Vi1 and pieces of position-corresponding-mind-and-body-state-associated information Vi2 based on arbitrary indexes. This allows the position-corresponding-emotion-evaluation system 8 to effectively use information on an emotion, while suppressing an increase in the processing load of hardware.

### [Fourth Embodiment]

### <Position-Corresponding-Emotion-Evaluation Device>

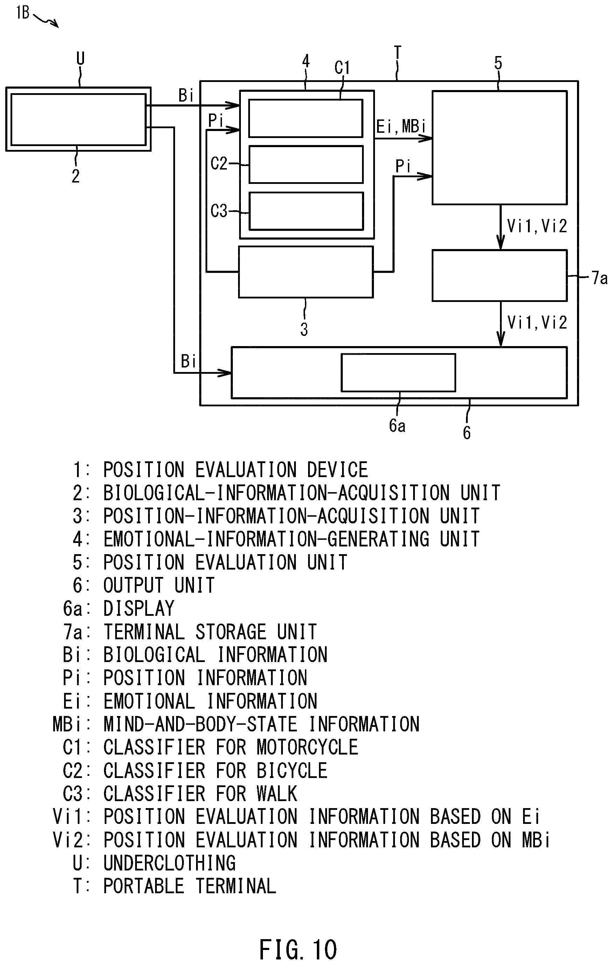

An overall configuration of a position-corresponding-emotion-evaluation device 1B according to a fourth embodiment of the present teaching is now described with reference to FIG. 10. FIG. 10 is a schematic view of an overall configuration of the position-corresponding-emotion-evaluation device 1B according to the fourth embodiment of the present teaching. The position-corresponding-emotion-evaluation device 1B according to the embodiment of the present teaching selects a classifier C appropriate for a means of transportation of a target person.

As shown in FIG. 10, the position-corresponding-emotion-evaluation device 1B includes the biological-information-acquisition unit 2, the position-information-acquisition unit 3, the emotional-information-generating unit 4, the position evaluation unit 5, the terminal storage unit 7a, and the output unit 6. The position-corresponding-emotion-evaluation device 1B is constituted of the wearable sensor provided in underclothing U, for example, and the portable terminal T. A target person wears the underclothing U that is the wearable sensor, and also carries a smartphone that is the portable terminal T.

The emotional-information-generating unit 4 that is a part of a control unit of the position-corresponding-emotion-evaluation device 1B has a classifier C1 for motorcycle, a classifier C2 for bicycle and a classifier C3 for walk that are criteria for generating emotional information Ei based on biological information Bi. The emotional-information-generating unit 4 acquires, per unit of time, biological information Bi on at least a heartbeat of the target person and triaxial acceleration that include information on time from the biological-information-acquisition unit 2. The emotional-information-generating unit 4 also acquires, per unit of time, position information Pi on a place where the target person is located that includes information on time, from the position-information-acquisition unit 3.

The emotional-information-generating unit 4 calculates a moving speed V of the target person based on the position information Pi of the target person. The emotional-information-generating unit 4 determines a means of transportation of the target person based on the calculated moving speed V of the target person. In a case where the moving speed V of the target person is equal to or higher than a reference value Vs, the emotional-information-generating unit 4 determines that the target person is moving by motorcycle. In a case where the moving speed V of the target person is lower than the reference value Vs, the emotional-information-generating unit 4 determines that the target person is moving by bicycle or on foot. In a case where the triaxial acceleration includes a certain proportion of leftward or rightward acceleration with respect to the moving direction of the target person, the emotional-information-generating unit 4 determines that the target person is riding on a bicycle that is a leaning vehicle.

The emotional-information-generating unit 4 selects a classifier in accordance with the means of transportation determined for the target person. In a case where the emotional-information-generating unit 4 determines that the target person is moving by motorcycle, the unit selects the classifier C1 for motorcycle. In a case where the emotional-information-generating unit 4 determines that the target person is moving by bicycle, the unit selects the classifier C2 for bicycle. In a case where the emotional-information-generating unit 4 determines that the target person is moving on foot, the unit selects the classifier C3 for walk. The emotional-information-generating unit 4 generates emotional information Ei and mind-and-body-state information MBi based on the acquired biological information Bi on at least a heartbeat of the target person, by using the selected classifier. The emotional-information-generating unit 4 generates different emotional information Ei based on the same biological information Bi by changing the classifier.

In a case where the emotional-information-generating unit 4 selects, for example, the classifier C1 for motorcycle, the unit selects "agitation" from among a plurality of emotions, based on a heart rate H1 that is biological information Bi on at least a heartbeat of the target person. Likewise, the emotional-information-generating unit 4 selects "tension" from among a plurality of mind and body states, based on the heart rate H1 that is the biological information Bi on at least a heartbeat of the target person.

In a case where the emotional-information-generating unit 4 selects, for example, the classifier C2 for bicycle, the unit selects "excitement" from among the plurality of emotions, based on the heart rate H1 that is the biological information Bi on at least a heartbeat of the target person. Likewise, the emotional-information-generating unit 4 selects "exaltation" from among the plurality of mind and body states, based on the heart rate H1 that is the biological information Bi on at least a heartbeat of the target person.

In a case where the emotional-information-generating unit 4 selects, for example, the classifier C3 for walk, the unit selects "unpleasantness" from among the plurality of emotions, based on the heart rate H1 that is the biological information Bi on at least a heartbeat of the target person. Likewise, the emotional-information-generating unit 4 selects "tension" from among the plurality of mind and body states, based on the heart rate H1 that is the biological information Bi on at least a heartbeat of the target person.

In this manner, in a case where the heart rate H1 is the same, but the means of transportation of the target person differs, the emotional-information-generating unit 4 generates different emotional information Ei and mind-and-body-state information MBi. The emotional-information-generating unit 4 may select a classifier based on information on a target person. In a case where heart rates and means of transportation are the same, but genders and ages, for example, that are information on target persons, differ, the emotional-information-generating unit 4 generates different emotional information Ei and mind-and-body-state information MBi.

The position evaluation unit 5 of the position-corresponding-emotion-evaluation device 1B generates position-corresponding-emotion-associated information Vi1 and position-corresponding-mind-and-body-state-associated information Vi2 based on position information Pi acquired by the position-information-acquisition unit 3, as well as emotional information Ei and mind-and-body-state information MBi acquired by the emotional-information-generating unit 4.

The output unit 6 of the position-corresponding-emotion-evaluation device 1B displays the emotion label EL at a position of the target person on the map information M, based on emotional information Ei associated with position information Pi, or position information Pi associated with emotional information Ei, extracted from position-corresponding-emotion-associated information Vi1, where the map information M has a scale, a scrolling speed and display items determined based on the moving speed V of the target person (see FIG. 8).

### <Control Flow>

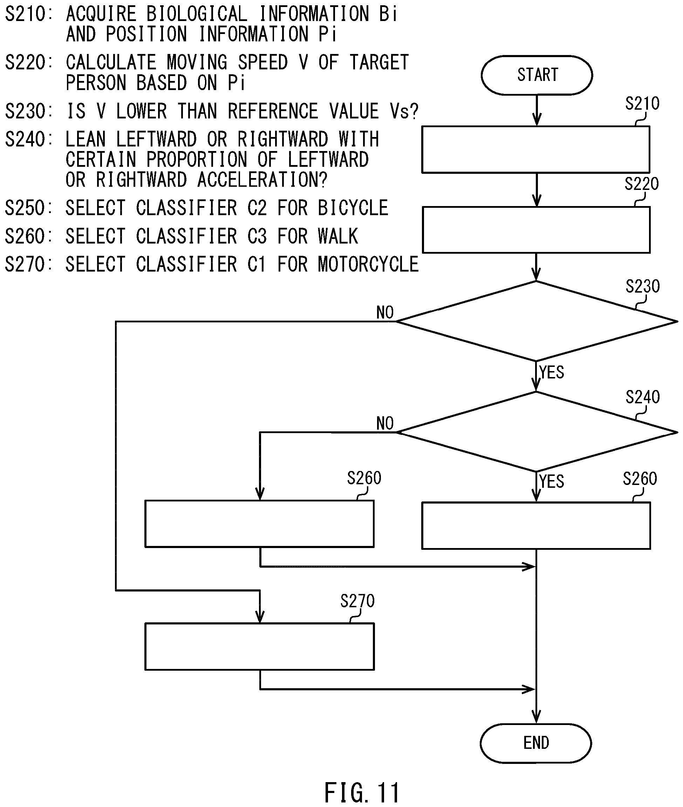

A control flow for selecting a classifier in the position-corresponding-emotion-evaluation device 1B is now described with reference to FIG. 11. FIG. 11 is a control flow diagram showing control of the position-corresponding-emotion-evaluation device 1B according to the fourth embodiment of the present teaching. It is to be noted that the position-corresponding-emotion-evaluation device 1B has already acquired a start signal for position-corresponding emotion evaluation.

As shown in FIG. 11, the biological-information-acquisition unit 2 of the position-corresponding-emotion-evaluation device 1B acquires, per unit of time, biological information Bi and position information Pi that include triaxial acceleration and time, for example at step S210.

At step S220, the emotional-information-generating unit 4 of the position-corresponding-emotion-evaluation device 1B calculates a moving speed V of a target person based on the position information Pi.

At step S230, the emotional-information-generating unit 4 determines whether the calculated moving speed V of the target person is lower than a reference value Vs or not. In a case where the moving speed V of the target person is lower than the reference value Vs, the emotional-information-generating unit 4 advances the flow to step S240. In a case where the moving speed V of the target person is equal to or higher than the reference value Vs, the emotional-information-generating unit 4 advances the flow to step S270.

At step S240, the emotional-information-generating unit 4 determines, based on the acquired triaxial acceleration, whether the triaxial acceleration includes a certain proportion of leftward or rightward acceleration that represents leftward or rightward leaning, with the moving direction of the target person as the forward direction. In a case where the target person leans leftward or rightward with a certain proportion of leftward or rightward acceleration, the emotional-information-generating unit 4 advances the flow to step S250. On the other hand, in a case where the target person does not lean leftward or rightward, without a certain proportion of leftward or rightward acceleration, the emotional-information-generating unit 4 advances the flow to step S260.

At step S250, the emotional-information-generating unit 4 determines that the target person is moving by bicycle and selects the classifier C2 for bicycle, then terminating the control for selecting a classifier.

At step S260, the emotional-information-generating unit 4 determines that the target person is moving on foot and selects the classifier C3 for walk, then terminating the control for selecting a classifier.

At step S270, the emotional-information-generating unit 4 determines that the target person is moving by motorcycle and selects the classifier C1 for motorcycle, then terminating the control for selecting a classifier.

The position-corresponding-emotion-evaluation device 1B thus configured generates emotional information Ei and mind-and-body-state information MBi by using different classifiers for different moving speeds V of the target person, based on the acquired triaxial acceleration of the target person and the acquired position information Pi on a place where the target person is located. That is, the position-corresponding-emotion-evaluation device 1B generates emotional information Ei and mind-and-body-state information MBi by using a classifier corresponding the means of transportation of the target person, among different classifiers for different means of transportation. The position-corresponding-emotion-evaluation device 1B can generate position-corresponding-emotion-associated information Vi1 and position-corresponding-mind-and-body-state-associated information Vi2, taking into consideration the difference in the means of transportation of the target person that is an external event affecting an emotion and a mind and body state of the target person by associating triaxial acceleration and position information Pi of the target person. This allows the position-corresponding-emotion-evaluation device 1B to effectively use information on an emotion, taking into consideration the influence of a moving speed V (a means of transportation) on an emotion of the target person, without the processing load applied to hardware.

### (Other Embodiments)

In the second embodiment described above, the emotional-information-generating unit 4 of the position-corresponding-emotion-evaluation device 1B selects a classifier in accordance with a means of transportation of a target person. Alternatively, the emotional-information-generating unit 4 may select a classifier by using time as an index, based on information on time included in, for example, information on a place where the target person is located. For example, the emotional-information-generating unit may have different classifiers corresponding to different seasons that are to be selected based on the information on time. The emotional-information-generating unit may have different classifiers corresponding to different time frames of a day, such as a classifier for morning, a classifier for afternoon, and a classifier for night.

In the fourth embodiment described above, the classifies of the emotional-information-generating unit 4 of the position-corresponding-emotion-evaluation device 1B include the classifier C1 for motorcycle, the classifier C2 for bicycle, and the classifier C3 for walk to be selected in accordance with a means of transportation of the target person. Alternatively, the classifiers may include, for example, a classifier for four-wheeled vehicle, a classifier for ship, and a classifier for train to correspond to different means of transportation of the target person. For example, the emotional-information-generating unit 4 may determine a means of transportation of the target person, not only from the moving speed V of the target person, but also from the map information M.

The position-corresponding-emotion-evaluation device 1B generates, for example, emotional information Ei that differs in accordance with seasons and time frames, by using, as an index, time included in acquired position information Pi on a place where the target person is located. That is, the position-corresponding-emotion-evaluation device 1B generates emotional information Ei and mind-and-body-state information MBi, taking into consideration a change in surrounding environment according to a difference in time that is an external event affecting an emotion and a mind and body state of the target person. The position-corresponding-emotion-evaluation device 1B associates emotional information Ei and position information Pi, and can thereby evaluate the position information Pi, with the emotional information Ei reflecting difference in season and time frame as an index, for example. The position-corresponding-emotion-evaluation device 1B also associates mind-and-body-state information MBi and position information Pi, and can thereby evaluate the position information Pi, with the mind-and-body-state information MBi reflecting difference in season and time frame as an index, for example. This allows the position-corresponding-emotion-evaluation device 1B to effectively use emotional information, taking into consideration the influence of seasons and time frames on an emotion, without the processing load applied to hardware.

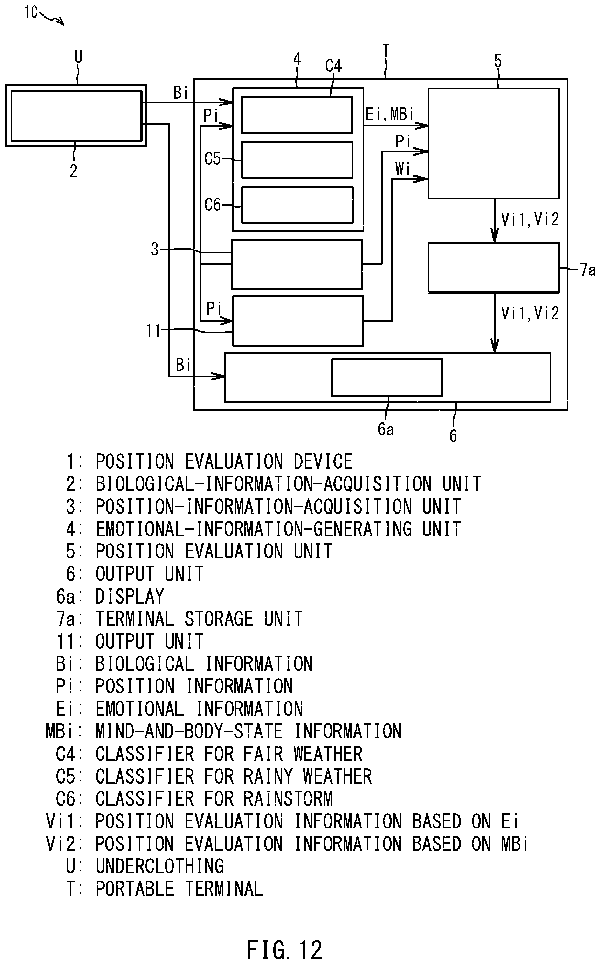

As shown in FIG. 12, in all of the embodiments described above, the position-corresponding-emotion-evaluation devices 1, 1A and 1B may include a weather-information-acquisition unit 12. FIG. 12 is a schematic view of an overall configuration of a position-corresponding-emotion-evaluation device 1C according to another embodiment of the present teaching. In this case, the emotional-information-generating unit 4 of the position-corresponding-emotion-evaluation device 1C has different classifiers corresponding to different weather conditions, such as temperature and weather. The emotional-information-generating unit has, for example, a classifier C4 for fair weather, a classifier C5 for rainy weather, and a classifier C6 for rainstorm. The emotional-information-generating unit 4 acquires weather information WEi on a place where a target person is located, such as temperature and weather. The emotional-information-generating unit 4 selects a classifier based on the weather information WEi. For example, in a case where the target person is located in a place where it is raining, the emotional-information-generating unit 4 selects the classifier C5 for rainy weather.

The position-corresponding-emotion-evaluation device 1C generates emotional information Ei and mind-and-body-state information MBi, taking into consideration weather conditions affecting an emotion and a mind and body state of the target person. The position-corresponding-emotion-evaluation device 1C associates position information Pi and emotional information Ei, and can thereby evaluate the position information Pi by the emotional information Ei reflecting weather information that is an external event affecting an emotion and a mind and body state of the target person. The position-corresponding-emotion-evaluation device 1C also associates position information Pi and mind-and-body-state information MBi, and can thereby evaluate the position information Pi by the mind-and-body-state information MBi reflecting weather information that is an external event affecting a mind and body state of the target person. This allows the position-corresponding-emotion-evaluation device 1C to effectively use information on an emotion, taking into consideration the influence of weather conditions on an emotion, without the processing load applied to hardware.

In all of the embodiments described above, the biological-information-acquisition unit 2 acquires biological information Bi on at least a heartbeat of a target person. However, the biological-information-acquisition unit is not limited to the configurations of the above-described embodiments. For example, the biological-information-acquisition unit may be configured to acquire information on the living body of the target person, other than the biological information Bi on the heartbeat of the target person, such as body surface temperature of the target person and triaxial acceleration of the target person.

In the respective embodiments described above, the emotional-information-generating unit 4 has at least one of a classifier for each different means of transportation of a target person, a classifier for each different weather condition, a classifier for each different time frame, or a classifier for each different information on a target person. However, the position-corresponding-emotion-evaluation devices are not limited to the configurations of the above-described embodiments. For example, the emotional-information-generating unit 4 may have classifiers that use geographic characteristics as an index.

In all of the embodiments described above, the classifiers of the emotional-information-generating unit 4 classify evaluation data D with a valence value E and an arousal value A as indexes, by using a pattern recognition model. However, the classifiers are not limited to the configuration in which the valence value E and the arousal value A are used as the indexes. For example, the classifiers may generate emotional information Ei on an emotion of a target person by using arbitrarily defined indexes concerning emotions.

In the second embodiment described above, the classifiers C of the emotional-information-generating unit 4 in the position-corresponding-emotion-evaluation device 1A classify evaluation data D by a pattern recognition model generated through machine leaning using training data. Alternatively, the classifiers may be configured to undergo further machine learning by acquiring feedback on the generated emotional information Ei from a target person. Thus, the classifiers are improved to have an appropriate pattern recognition model for the target person who uses the position-corresponding-emotion-evaluation device, through repeated use of the device.

The embodiments of the present teaching have been described above, but the above-described embodiments are merely illustrative examples of preferred embodiments of the present teaching. Therefore, the present teaching is not limited to the above-described embodiments and the above-described embodiments can be appropriately modified and implemented without departing from the gist of the teaching.

### REFERENCE SIGNS LIST

- 1, 1A, 1B, 1C: position-corresponding-emotion-evaluation device
- 2: biological-information-acquisition unit
- 3: position-information-acquisition unit
- 4: emotional-information-generating unit
- 5: position evaluation unit
- 6: output unit
- 6a: display
- 7: storage unit
- 7a: terminal storage unit
- 8: position-corresponding-emotion-evaluation system
- C: classifier
- Bi: biological information on heartbeat of target person
- Pi: position information on place where target person is located
- Ei: emotional information on emotion of target person
- Vi1: position-corresponding-emotion-associated information
- Vi2: position-corresponding-mind-and-body-state-associated information

## Claims

1. A position-corresponding-emotion-evaluation device, comprising:
a storage unit configured to store biological information on at least a heartbeat of a target person, and position information on a position where the biological information has been measured; and
a control unit configured to acquire, and store in the storage unit, the biological information and the position information, have a criterion for generating emotional information on an emotion based on the biological information, generate the emotional information based on the biological information, based on the criterion, and output the position information and the emotional information,
wherein
the control unit is configured to
generate position-corresponding-emotion-associated information in which the position information on the position where the biological information has been measured, and the emotional information on the emotion of the target person generated using the biological information measured at the position, are associated, and
output the emotional information associated with the position information, or the position information associated with the emotional information, from the generated position-corresponding-emotion-associated information.

2. The position-corresponding-emotion-evaluation device according to claim 1, wherein
the emotion includes a plurality of types of emotions, and
the emotional information is emotional information on an emotion selected from the plurality of the types of the emotions.

3. The position-corresponding-emotion-evaluation device according to claim 1 or 2, wherein
the emotional information includes information on degree of an emotion.

4. The position-corresponding-emotion-evaluation device according to any one of claims 1 to 3, wherein
the control unit is configured to
calculate a moving speed of the target person based on the position information and time at which the position information has been acquired, and generate the position-corresponding-emotion-associated information based on the calculated moving speed, and
output the emotional information associated with the position information, or the position information associated with the emotional information, from the position-corresponding-emotion-associated information generated based on the moving speed.

5. The position-corresponding-emotion-evaluation device according to any one of claims 1 to 4, wherein
the control unit is configured to
generate the position-corresponding-emotion-associated information based on the time at which the position information has been acquired, and
output the emotional information associated with the position information, or the position information associated with the emotional information, from the position-corresponding-emotion-associated information generated based on the time.

6. The position-corresponding-emotion-evaluation device according to any one of claims 1 to 5, wherein
the control unit is configured to
acquire weather information based on the position information,
generate the position-corresponding-emotion-associated information based on the weather information, and
output the emotional information associated with the position information, or the position information associated with the emotional information, from the position-corresponding-emotion-associated information generated based on the weather information.

7. The position-corresponding-emotion-evaluation device according to any one of claims 1 to 6, wherein the control unit is configured to output the emotional information in form of an emotion label that is obtained by converting the emotional information into at least one of a numerical value, a mark, a figure or a color.

8. A position-corresponding-emotion-evaluation system, comprising:
a storage unit configured to store pieces of biological information on at least heartbeats of target persons, and pieces of position information on positions where the pieces of the biological information have been measured, the pieces of the biological information and the pieces of the position information having been acquired from the target persons; and
a control unit configured to acquire, and store in the storage unit, the pieces of the biological information and the pieces of the position information, have a criterion for generating pieces of emotional information on emotions based on the pieces of the biological information, generate the pieces of the emotional information based on the pieces of the biological information, based on the criterion, and output the pieces of the position information and the pieces of the emotional information,
wherein
the control unit is configured to
generate pieces of position-corresponding-emotion-associated information in which the pieces of the position information on the positions where the pieces of the biological information have been measured, and the pieces of the emotional information on the emotions of the target persons generated using the pieces of the biological information measured at the positions, are associated, and
output the pieces of the emotional information associated with the pieces of the position information, or the pieces of the position information associated with the pieces of the emotional information, from the generated pieces of the position-corresponding-emotion-associated information.
